# EUROPEAN PATENT SPECIFICATION

(11) **EP 4 337 079 B1**
(45) Date of publication and mention of the grant of the patent: **22.10.2025**
(21) Application number: 22726949.5
(22) Date of filing: 11.05.2022
(51) Int. Cl.: A61B 1/00, A61B 1/005

(54) **MOTORIZED CONTROL FOR MEDICAL DEVICE**
MOTORISIERTE STEUERUNG FÜR EINE MEDIZINISCHE VORRICHTUNG
COMMANDE MOTORISÉE POUR DISPOSITIF MÉDICAL

(30) Priority: 13.05.2021 US 202163187988 P
(43) Date of publication of application: 20.03.2024
(73) Proprietor: Boston Scientific Scimed, Inc., Maple Grove, MN 55311 (US); Worcester Polytechnic Institute, Worcester, MA 01609 (US)
(72) Inventor: GU, Anne, Brighton, Massachusetts 02135 (US); NYCZ, Christopher J., Holden, Massachusetts 01520 (US); TAVAKKOLMOGHADDAM, Farid, Worcester, Massachusetts 01605 (US)
(74) Representative: Vossius & Partner Patentanwälte Rechtsanwälte mbB
(86) International application number: PCT/US2022/028757
(87) International publication number: WO 2022/240980

(56) References cited:
- DE-U1- 202010 009 234
- US-A- 4 982 725
- US-A1- 2021 045 626

## Description

### CROSS REFERENCE TO RELATED APPLICATIONS

The present application claims the benefit of and priority to US Provisional Patent Application No. 63/187,988, filed May 13, 2021.

### TECHNICAL FIELD

The disclosure is directed to a motorized control for a medical device. More particularly, the disclosure is directed to medical device, such as an endoscope or steerable catheter, having a motor control assembly configured to control movement of an articulating distal tip of the elongate shaft of the medical device.

### BACKGROUND

Medical devices, such as steerable/deflectable endoscopes and/or catheters, may be used to perform various diagnostic and/or treatment procedures. Different procedures may require different devices and/or different physical actions by the practitioner. Of the known medical devices, systems, and methods, each has certain advantages and disadvantages. There is an ongoing need to provide alternative medical devices and systems, including controls for manipulating and maneuvering such medical devices.

### SUMMARY

According to the invention, a steerable medical device comprises a handle and an elongate shaft extending distally from the handle to a distal tip, a motor control assembly including a motor control housing configured to detachably interface with the handle, wherein the motor control assembly includes a first motor disposed within the motor control housing and a first gear assembly disposed within the motor control housing, and a first drive axle configured to engage the first gear assembly with a first deflection mechanism disposed within the handle, the first deflection mechanism being configured to deflect the distal tip in a first plane.

In another example, an endoscopic system may comprise an endoscope including a handle and an elongate shaft extending distally from the handle to a distal tip, a motor control assembly including a motor control housing configured to detachably interface with the handle, wherein the motor control assembly includes a first motor disposed within the motor control housing and a first gear assembly disposed within the motor control housing, and a first drive axle configured to engage the first gear assembly with a first deflection mechanism disposed within the handle, the first deflection mechanism being configured to deflect the distal tip in a first plane.

In addition or alternatively to any example disclosed herein, the motor control assembly further includes a second motor disposed within the motor control housing and a second gear assembly disposed within the motor control housing.

In addition or alternatively to any example disclosed herein, the medical device and/or endoscopic system may include a second drive axle configured to engage the second gear assembly with a second deflection mechanism disposed within the handle, the second deflection mechanism being configured to deflect the distal tip in a second plane different from the first plane.

In addition or alternatively to any example disclosed herein, the first drive axle is coaxial with the second drive axle.

In addition or alternatively to any example disclosed herein, the first drive axle is parallel with the second drive axle.

In addition or alternatively to any example disclosed herein, the first drive axle is laterally offset from the second drive axle.

In addition or alternatively to any example disclosed herein, the motor control assembly includes a joystick control configured to operate the first motor.

In addition or alternatively to any example disclosed herein, the motor control assembly includes a joystick control configured to operate the first motor and the second motor.

In addition or alternatively to any example disclosed herein, the joystick control is configured to operate the first motor and the second motor simultaneously.

In addition or alternatively to any example disclosed herein, the medical device and/or endoscopic system may comprise a homing feature configured to return the distal tip to a home position when activated and/or a speed control feature configured to control speed and/or responsiveness of movement of the distal tip.

In another example, a medical device may comprise a handle and an elongate shaft extending distally from the handle to a distal tip; a motor control assembly including a motor control housing configured to detachably interface with the handle; wherein the motor control assembly includes: a first motor disposed within the motor control housing, a first gear assembly disposed within the motor control housing, a second motor disposed within the motor control housing, and a second gear assembly disposed within the motor control housing; and a first drive axle coaxially disposed within a lumen of a second drive axle. The first drive axle may be configured to engage the first gear assembly with a first deflection mechanism disposed within the handle, the first deflection mechanism being configured to deflect the distal tip in a first plane. The second drive axle may be configured to engage the second gear assembly with a second deflection mechanism disposed within the handle, the second deflection mechanism being configured to deflect the distal tip in a second plane different from the first plane.

In another example, an endoscopic system may comprise an endoscope including a handle and an elongate shaft extending distally from the handle to a distal tip; a motor control assembly including a motor control housing configured to detachably interface with the handle; wherein the motor control assembly includes: a first motor disposed within the motor control housing, a first gear assembly disposed within the motor control housing, a second motor disposed within the motor control housing, and a second gear assembly disposed within the motor control housing; and a first drive axle coaxially disposed within a lumen of a second drive axle. The first drive axle may be configured to engage the first gear assembly with a first deflection mechanism disposed within the handle, the first deflection mechanism being configured to deflect the distal tip in a first plane. The second drive axle may be configured to engage the second gear assembly with a second deflection mechanism disposed within the handle, the second deflection mechanism being configured to deflect the distal tip in a second plane different from the first plane.

In addition or alternatively to any example disclosed herein, the first drive axle is fixedly secured to the first deflection mechanism.

In addition or alternatively to any example disclosed herein, the first drive axle is configured to slidably engage the first gear assembly.

In addition or alternatively to any example disclosed herein, the second drive axle is fixedly secured to the second deflection mechanism.

In addition or alternatively to any example disclosed herein, the second drive axle is configured to slidably engage the second gear assembly.

In another example, a medical device may include a handle, an elongate shaft extending distally from the handle to a distal tip, and an umbilicus extending from the handle to a proximal connector having an optical and/or electrical connection, wherein the proximal connector includes a first deflection mechanism disposed therein, the first deflection mechanism being configured to deflect the distal tip in a first plane.

In another example, an endoscopic system may comprise an endoscope including: a handle, an elongate shaft extending distally from the handle to a distal tip, and an umbilicus extending from the handle to a proximal connector having an optical and/or electrical connection, wherein the proximal connector includes a first deflection mechanism disposed therein, the first deflection mechanism being configured to deflect the distal tip in a first plane.

In addition or alternatively to any example disclosed herein, the medical device and/or endoscopic system may comprise a controller including a controller housing and a first motor disposed within the controller housing. The proximal connector may be configured to releasably couple to the controller. The first motor may be configured to operatively engage the first deflection mechanism when the proximal connector is releasably coupled to the controller.

In addition or alternatively to any example disclosed herein, the proximal connector includes a second deflection mechanism disposed therein, the second deflection mechanism being configured to deflect the distal tip in a second plane different from the first plane. The controller includes a second motor disposed within the controller housing, the second motor being configured to operatively engage the second deflection mechanism when the proximal connector is releasably coupled to the controller.

In addition or alternatively to any example disclosed herein, the handle includes at least one input mechanism configured to control deflection of the distal tip in the first plane.

In addition or alternatively to any example disclosed herein, the at least one input mechanism is in electronic communication with the controller when the proximal connector is releasably coupled to the controller.

The above summary of some embodiments, aspects, and/or examples is not intended to describe each embodiment or every implementation of the present disclosure. The figures and the detailed description which follows more particularly exemplify these embodiments.

### BRIEF DESCRIPTION OF THE DRAWINGS

Embodiments of the disclosure may be more completely understood in consideration of the following detailed description in connection with the accompanying drawings, in which:
FIG. 1 illustrates selected aspects of a medical device, depicted as an endoscopic system;
FIG. 2 illustrates selected aspects of the medical device, namely the endoscopic system, of FIG. 1;
FIG. 3 illustrates selected aspects of the medical device, namely the endoscopic system, of FIG. 1;
FIG. 4 is a partial section view illustrating selected aspects of the medical device, namely the endoscopic system, of FIG. 1;
FIG. 5 is a partial section view illustrating selected aspects of the medical device, namely the endoscopic system, of FIG. 1;
FIG. 6 is a partial section view illustrating selected aspects of the medical device, namely the endoscopic system, of FIG. 1;
FIGS. 7-8 illustrate selected aspects of a first configuration of the medical device, namely the endoscopic system, of FIG. 1;
FIGS. 9-10 illustrate selected aspects of a second configuration of the medical device, namely the endoscopic system, of FIG. 1;
FIG. 11 illustrates selected aspects of an alternative medical device, depicted as an endoscopic system;
FIGS. 12-14 illustrate selected aspects of the medical device, namely the endoscopic system, of FIG. 11;
FIGS. 15-17 illustrate aspects of an alternative medical device, depicted as an endoscopic system; and
FIG. 18 schematically illustrates aspects of an alternative medical device, depicted as an endoscopic system.

While the embodiments of the present disclosure are amenable to various modifications and alternative forms, specifics thereof have been shown by way of example in the drawings and will be described in detail. It should be understood, however, that the intention is not to limit aspects of the present disclosure to the particular embodiments described.

### DETAILED DESCRIPTION

The following description should be read with reference to the drawings, which are not necessarily to scale, wherein like reference numerals indicate like elements throughout the several views. The detailed description and drawings are intended to illustrate but not limit the claims. Those skilled in the art will recognize that the various elements described and/or shown may be arranged in various combinations and configurations without departing from the scope of the disclosure. The detailed description and drawings illustrate example embodiments. However, in the interest of clarity and ease of understanding, while every feature and/or element may not be shown in each drawing, the feature(s) and/or element(s) may be understood to be present regardless, unless otherwise specified.

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5).

Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the embodiments of the present disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "retract", variants thereof, and the like, may be generally considered with respect to the positioning, direction, and/or operation of various elements relative to a user of the device, wherein "proximal" and "retract" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to affect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

Some medical/surgical procedures - for example: kidney stone management, ERCP (endoscopic retrograde cholangiopancreatography), pulmonary biopsy, colonoscopy, bladder mapping, cardiac mapping, cardiac valve replacement and/or repair, and others - may require navigation of an elongate shaft of a medical device through tortuous anatomy to position the elongate shaft, such as the distal tip of the elongate shaft, of the medical device(s) at a specific location and/or orientation. Some of these procedures may be long and/or may involve difficult physical actions that may lead to physician fatigue and/or musculoskeletal injury. In some medical/surgical procedures, the physician may be at risk of work-related strain due to repetitive motions, prolonged awkward posture(s), high forces, contact stress, and/or vibration. For example, some physicians may be at risk to develop De Quervain's tenosynovitis (swelling and pain at the base of the thumb), carpal tunnel syndrome, ganglion cysts, "trigger finger", and/or other conditions. In some instances, a physician's hand size may negatively affect his or her ability to control the medical device and/or perform medical/surgical procedures. The current disclosure relates to features that may reduce and/or eliminate physician fatigue resulting from the procedure(s), may improve usability and/or ergonomics of the medical device for varying hand sizes, may provide more precision and/or more precise movements and stability when using the medical device, and may make using the medical device and/or performing certain procedures easier to learn.

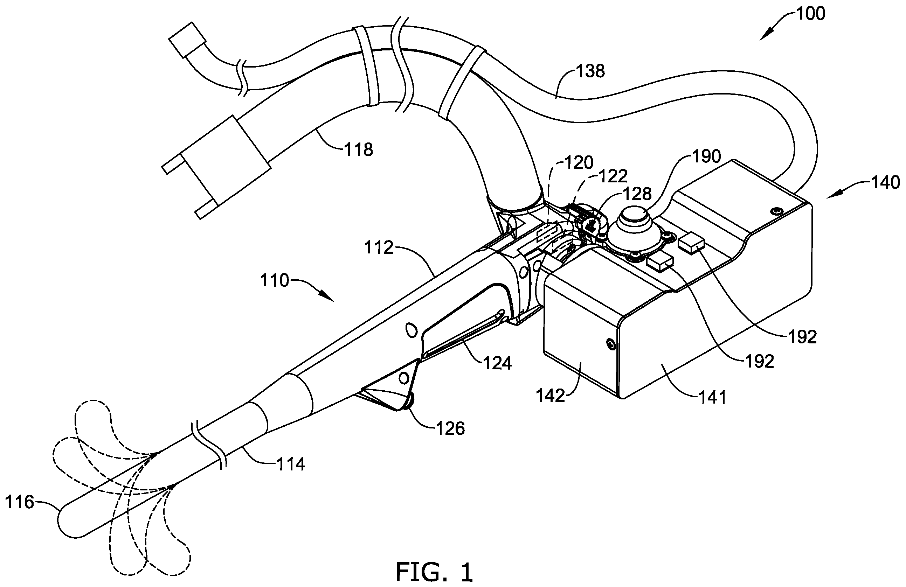

FIGS. 1-6 illustrate selected aspects of an exemplary medical device, depicted as an endoscopic system 100, according to the disclosure. In some embodiments, the endoscopic system 100 may include an endoscope 110. The endoscope 110 may be specific to a particular endoscopic procedure, such as, e.g., a ureteroscope, a cystoscope, a nephroscope, a duodenoscope, etc., or may be a general-purpose device suitable for a wide variety of procedures. According to the invention, the endoscope 110 includes a handle 112 and an elongate shaft 114 extending distally from the handle 112 to a distal tip 116, wherein the handle 112 includes a first deflection mechanism 120 configured to deflect and/or articulate the distal tip 116 of the elongate shaft 114 in a first plane and a second deflection mechanism 122 configured to deflect and/or articulate the distal tip 116 of the elongate shaft 114 in a second plane different from the first plane. In some embodiments, the first plane may be oriented at a non-zero angle to the second plane. In some embodiments, the first plane may be oriented perpendicular to the second plane. Other configurations are also contemplated. Although depicted as an endoscopic system 100 including an endoscope 110, it is noted and understood that features, components, and/or functionality described herein may be incorporated into another medical device, such as a steerable catheter or other medical device having one or more deflection mechanisms for controlling deflection and/or articulation of a distal tip of the elongate shaft of the medical device to facilitate navigation of the elongate shaft through the anatomy of a patient. Accordingly, the described components such as the handle, elongate shaft, deflection mechanisms, motor control assembly, and other components may be associated with another medical device having a deflectable/steerable distal tip of an elongate shaft, as desired.

In some embodiments, the first deflection mechanism 120 may include a first pulley or rotating member disposed within the handle 112 and operatively connected to the distal tip 116. In some embodiments, one or more first cables, wires, or other filaments may be engaged with and/or connected to the first pulley within the handle 112. In some embodiments, the one or more first cables, wires, or filaments may be engaged with and/or connected to the distal tip 116, such that tension applied to the one or more first cables, wires, or filaments by the first pulley deflects and/or articulates the distal tip 116 in the first plane. In some embodiments, the second deflection mechanism 122 may include a second pulley or rotating member disposed within the handle 112 and operatively connected to the distal tip 116. In some embodiments, one or more second cables, wires, or other filaments may be engaged with and/or connected to the second pulley within the handle 112. In some embodiments, the one or more second cables, wires, or filaments may be engaged with and/or connected to the distal tip 116, such that tension applied to the one or more second cables, wires, or filaments by the second pulley deflects and/or articulates the distal tip 116 in the second plane. Other configurations are also contemplated.

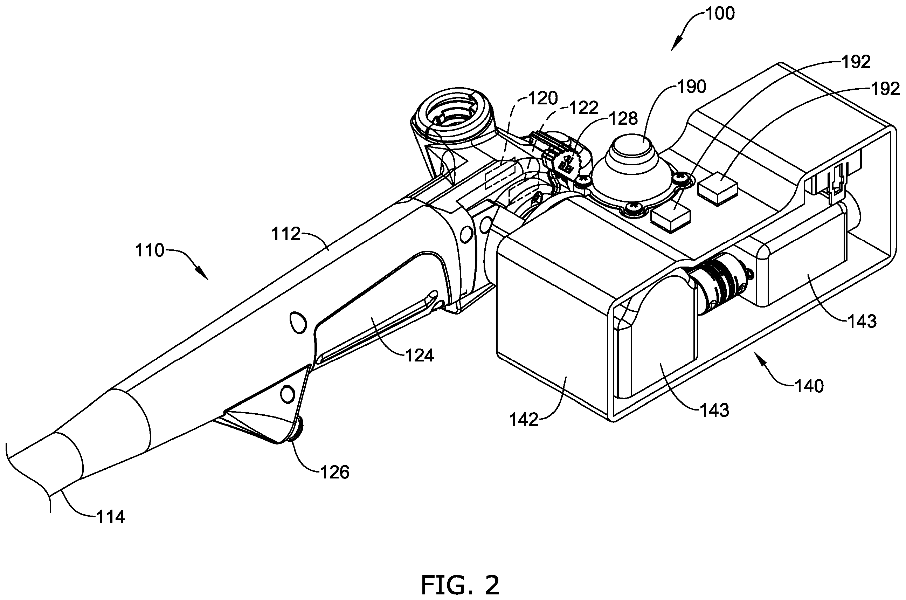

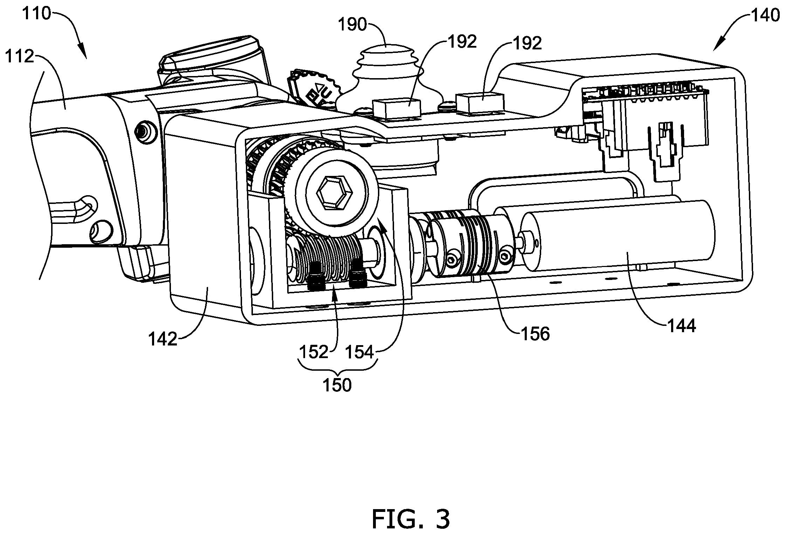

According to the invention, the endoscopic system 100 includes a motor control assembly 140 including a motor control housing 142 configured to detachably interface with the handle 112 of the endoscope 110. In some embodiments, the motor control housing 142 may include a removable cover 141 (e.g., FIG. 1), wherein internal components of the motor control assembly 140 may be accessed after removing the removable cover 141, as seen in FIG. 2. Additionally, in some embodiments, the motor control assembly 140 may include one or more internal covers 143 disposed within the motor control housing 142 that may protect certain components and/or groups of components from each other, contamination, etc. In some embodiments, the one or more internal covers 143 may provide structural support for selected internal components. In FIG. 3, the one or more internal covers 143 have been removed to facilitate discussion related to selected internal components of the motor control assembly 140. It shall be understood that the presence and/or use of all, some, or any of the one or more internal covers 143 is optional and is not required.

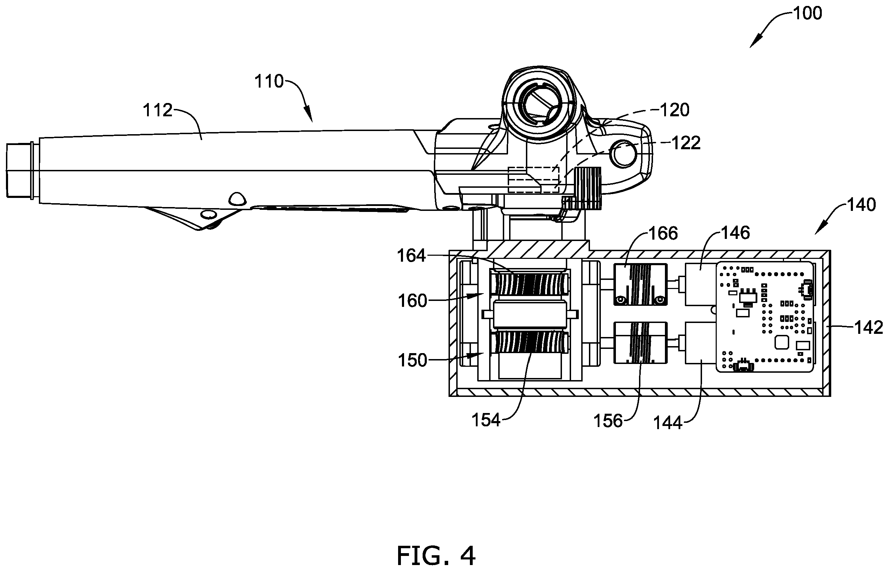

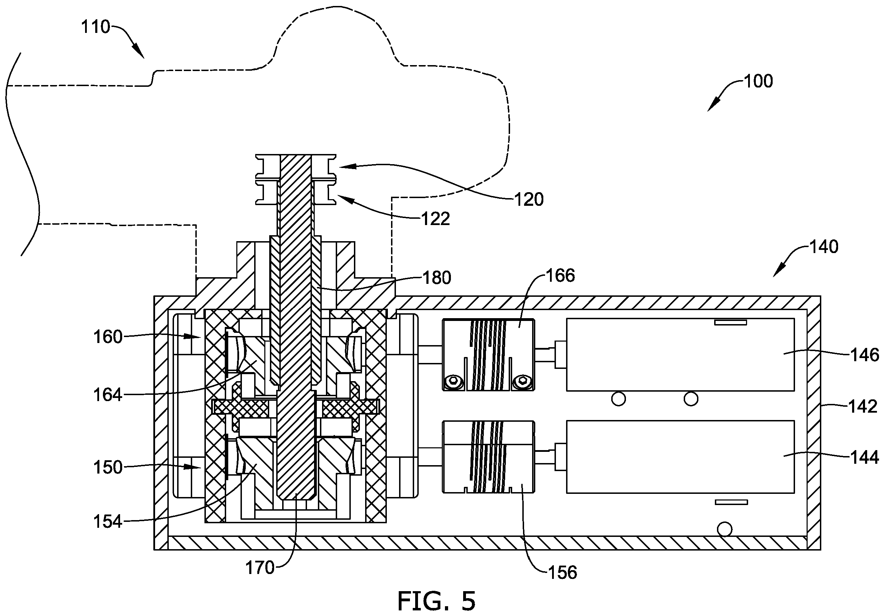

In some embodiments, the motor control assembly 140 may include at least one motor (e.g., FIGS. 3-5) disposed within the motor control housing 142. According to the invention, the at least one motor includes a first motor 144 disposed within the motor control housing 142, as seen in FIG. 3. In some embodiments, the at least one motor may include a second motor 146 disposed within the motor control housing 142, as seen in FIGS. 4 and 5. In some embodiments, the at least one motor may include the first motor 144 disposed within the motor control housing 142 and the second motor 146 disposed within the motor control housing 142. In some embodiments, for each controlled degree of freedom of the distal tip 116, an individual motor may be added to the at least one motor.

The at least one motor may be electric and may be brushed or brushless DC motors. The at least one motor may provide the user partial force assistance for deflecting the distal tip 116, from 0% to 100% force assistance, thereby allowing for fully manual control, assisted control with reduced lever/knob force, or fully actuated control. Each motor may be attached to a rotary encoder which provides a relative position of said motor to the processor unit. In some embodiments, the at least one motor may be controller by a central processing unit (CPU), a microprocessor, and/or a combination thereof. In some embodiments, additional inputs for the CPU, the microprocessor, and/or the combination thereof may include components of the user interface, tactile feedback electronics, etc.

In some embodiments, the motor control assembly 140 may include a user input mechanism. In some embodiments, the user input mechanism may include one or more of a joystick control, a scroll wheel, a knob, a slider, a keypad, a touch screen interface or control, a voice interface or control, etc. Returning briefly to FIGS. 1-3, in some embodiments, the motor control assembly 140 may include a joystick control 190 configured to operate the at least one motor disposed within the motor control housing 142. In some embodiments, the joystick control 190 may be configured to operate the first motor 144. In some embodiments, the joystick control 190 may be configured to operate the second motor 146. In some embodiments, the joystick control 190 may be configured to operate the first motor 144 and the second motor 146. In some embodiments, the joystick control 190 may be configured to operate the first motor 144 and the second motor 146 simultaneously. In some embodiments, the joystick control 190 may be configured to operate the first motor 144 and the second motor 146 independently of each other. Other configurations are also contemplated.

In some embodiments, the motor control assembly 140 may include a homing feature configured to return the distal tip 116 to a home position (e.g., to a straightened and/or non-deflected configuration) and/or a speed control feature configured to control speed and/or responsiveness of movement of the distal tip 116. In some embodiments, the homing feature and/or the speed control feature may each include an input mechanism. For example, the input mechanism may be one or more buttons, a voice interface and/or control, a gesture interface and/or control, or other suitable means of providing input to the motor control assembly 140 and/or the user interface.

In some embodiments, the motor control assembly 140 may include one or more buttons 192. In some embodiments, the one or more buttons 192 may include a first button (e.g., a home button) configured to return the distal tip 116 to the home position (e.g., to the straightened configuration), wherein activation of the first button automatically actuates the at least one motor to move the distal tip 116 to the home position. In some embodiments, the one or more buttons 192 may include a second button that is user configurable to retain a saved position or configuration, wherein activation of the second button automatically actuates the at least one motor to move the distal tip 116 to the saved position and/or configuration. Additional buttons and/or other configurations are also contemplated. Additionally, while the one or more buttons 192 are discussed herein by way of example, the motor control assembly 140 is not limited to the use of physical buttons and may include other input mechanisms.

In some embodiments, the motor control assembly 140 may include a self-homing feature and/or procedure. In one example, upon connecting the motor control assembly 140 to the handle 112, a homing program and/or algorithm is activated and/or run for the first motor 144 and the second motor 146 (where present) sequentially (e.g., the procedure is done on the first motor 144 and then is done on the second motor 146). In some instances, the homing program and/or algorithm is automatically initiated when the motor control assembly 140 is connected to the handle 112. In other instances, the user may push a button, or otherwise manually initiate the homing program and/or algorithm after the motor control assembly 140 is connected to the handle 112. The first motor 144 may start rotating in a first direction while the current is being monitored by the homing program and/or algorithm. Upon registering, encountering, and/or identifying a sudden increase in the amount of current drawn by the first motor 144, a location of an encoder is saved as a checkpoint for an upper limit or a lower limit. The first motor 144 then starts turning in a second direction opposite the first direction and again upon registering, encountering, and/or identifying a sudden increase in the amount of current drawn by the first motor 144, the value of the encoder is saved as a second checkpoint for the other limit of the motion range for that axis and/or the first motor 144. After determining the upper limit and the lower limit of the first motor 144 (and finding their corresponding values in terms of encoder values) the algorithm may divide the number of ticks between the upper limit and the lower limit by two. The corresponding encoder value would be the middle of the motion range or the home position for that axis and/or the first motor 144.

The same procedure is then repeated for the second motor 146, wherein the second motor 146 may start rotating in the first direction while the current is being monitored by the homing program and/or algorithm. Upon registering, encountering, and/or identifying a sudden increase in the amount of current drawn by the second motor 146, a location of an encoder is saved as a checkpoint for an upper limit or a lower limit. The second motor 146 then starts turning in the second direction opposite the first direction and again upon registering, encountering, and/or identifying a sudden increase in the amount of current drawn by the second motor 146, the value of the encoder is saved as a second checkpoint for the other limit of the motion range for that axis and/or the second motor 146. After determining the upper limit and the lower limit of the second motor 146 (and finding their corresponding values in terms of encoder values) the algorithm may divide the number of ticks between the upper limit and the lower limit by two. The corresponding encoder value would be the middle of the motion range or the home position for that axis and/or the second motor 146.

In another example, the homing feature and/or procedure may be manually activated after engaging the motor control assembly 140 to the handle 112 of the endoscope 110. Other configurations are also contemplated.

In some embodiments, the joystick control 190 may be configured to control movement of the distal tip 116. In some embodiments, the joystick control 190 may be configured to control a speed at which the distal tip 116 moves and/or responsiveness of the joystick control 190. In some embodiments, the motor control assembly 140 may include a speed change control button. In some embodiments, the speed change control button may be built into and/or may be integrated into the joystick control 190. For example, in some embodiments, pressing axially on the joystick control 190 toward the motor control housing 142 may actuate the speed change control button. The speed change control feature and/or the speed change control button may actuate and/or cycle through a plurality of speed settings to permit at least some degree of customization over the speed and/or responsiveness of movement of the distal tip 116. In some embodiments, the plurality of speed settings may include at least a high-speed setting which permits faster control and/or faster movement of the distal tip 116 and a low speed setting which permits finer control and/or slower movement of the distal tip 116. In some embodiments, the plurality of speed settings may further include a medium speed setting and/or other speed settings (e.g., medium-high, medium-low, extra low, etc.).

In some embodiments, the motor control housing 142, the speed change control feature, the speed change control button, and/or the plurality of speed settings may be user customizable. In some embodiments, the user may be able to set speed mode(s), select and/or set desired speed(s), and/or set or save the speed mode(s) and/or desired speed(s). In some embodiments, the speed change control button may be depressed and held to set or save the speed mode(s) and/or desired speed(s). In some embodiments, the motor control housing 142 and/or the user interface may include additional buttons, a keypad, a scroll wheel, a dial, a touch interface, or other input mechanism for setting and/or adjusting the speed mode(s) and/or desired speed(s). These are only examples, and other configurations are also contemplated.

In some embodiments, other control mechanisms may be used in place of the joystick control 190. In some embodiments, the motor control assembly 140 may include a keypad. In some embodiments, the motor control assembly 140 may include a scroll wheel. In some embodiments, the motor control assembly 140 may include a touch screen. Other control configurations are also contemplated.

In some embodiments, the distal tip 116 may include a camera and may, for example, have deflection and/or articulation capabilities in one or more directions for viewing patient anatomy. In some embodiments, the endoscope 110 may be a duodenoscope such as an Exalt^{™} Model D scope. However, other medical devices, such as another endoscope (e.g., a ureteroscope, etc.) or related system, (e.g., Lithovue^{™}, SpyScope^{™} DS, SpyGlass^{™} DS, etc.) may be used in addition to or in place of a duodenoscope and/or the endoscope 110. In some embodiments, the endoscope 110 may be configured to deliver fluid from a fluid management system to a treatment site via the elongate shaft 114. The elongate shaft 114 may include one or more working lumens for receiving a flow of fluid and/or other medical devices therethrough. In some embodiments, the endoscope 110 may be connected to the fluid management system via one or more supply line(s).

In some embodiments, the handle 112 of the endoscope 110 may include a plurality of elements configured to facilitate the endoscopic procedure. In some embodiments, an umbilicus 118 extends from the handle 112 and is configured for attachment to an electronic device (not pictured) such as e.g. a computer system, a console, a microcontroller, etc. for providing power, analyzing endoscopic data, controlling the endoscopic intervention, and/or performing other functions. In some embodiments, the electronic device to which the umbilicus 118 is connected may have functionality for recognizing and exchanging data with other endoscopic accessories. The handle 112 may include a grip area 124 for the operating physician to grasp while performing the endoscopic procedure. In some embodiments, the handle 112 may include a side port 126 in communication with the one or more working lumens of the elongate shaft 114 and/or the endoscope 110.

In some embodiments, a motor control umbilicus 138 may extend from the motor control housing 142 and may be configured for attachment to an electronic device, which may be the same electronic device that the umbilicus 118 is attached to or may be a different electronic device as desired, for providing power, controlling endoscopic intervention and/or the motor control assembly, and/or other functions. In some embodiments, the motor control umbilicus 138 may be secured to the umbilicus 118. In some embodiments, the motor control umbilicus 138 may be releasably and/or removably secured to the umbilicus 118. In some embodiments, the motor control umbilicus 138 may be secured to the umbilicus 118 with a hook and loop closure device (e.g., Velcro^{™}), tape, a wire tie, or other securement apparatus.

In some embodiments, the endoscope 110 may be in electronic communication with a workstation via a wired connection (e.g., the umbilicus 118). In some embodiments, the workstation may include a touch panel computer, an interface box for receiving the wired connection (e.g., the umbilicus 118), a cart, and a power supply, among other features. In some embodiments, the interface box may be configured with a wired or wireless communication connection with the controller of the fluid management system. The touch panel computer may include at least a display screen and an image processor. In some embodiments, the workstation may be a multi-use component (e.g., used for more than one procedure) while the endoscope 110 may be a single use device, although this is not required. In some embodiments, the workstation may be omitted and the endoscope 110 may be electronically coupled directly to the controller of a fluid management system, where used.

In some embodiments, the handle 112 may include at least one communication interface for attaching accessory devices. In some embodiments, the handle 112 may include Universal Serial Bus type-C (USB-C) ports, Universal Serial Bus (USB) ports, ethernet ports, and/or other types of ports. In some embodiments, more, less, and/or other communication interfaces of various types, including, for example, custom interfaces, may be used. In some embodiments, the handle 112 has only one communication interface but may be connectable to e.g. a USB hub with multiple ports for connecting multiple accessories. In some embodiments, the at least one communication interface may provide power to the accessory device(s) in addition to exchanging data therewith. Thus, the accessory device(s) need not have separate cables running to a connected electronic device or a battery that adds additional weight to the handle 112. In some embodiments, the accessory device(s) may be uniquely associated with the endoscope 110 and recognized by the electronic device through "plug and play" functionality without any user setup required.

In some embodiments, the endoscope 110 may include one or more sensors proximate the distal tip 116 and/or the distal end of the elongate shaft 114. For example, the endoscope 110 may include a pressure sensor at the distal tip 116 of the elongate shaft 114 to measure intracavity pressure within the treatment site. The endoscope 110 may also include other sensors such as, for example, a temperature sensor, a Fiber Bragg grating optical fiber to detect stresses, and/or an antenna or electromagnetic sensor (e.g., a position sensor). In some embodiments, the distal tip 116 and/or the distal end of the endoscope 110 may also include at least one camera to provide a visual feed to the user on the display screen of the touch panel computer. In another embodiment, the endoscope 110 may include two cameras having different communications requirements or protocols so that different information may be relayed to the user by each camera. When so provided, the user may switch back and forth between the cameras at will through the touch screen interface and/or the touch panel computer. While not explicitly shown, the elongate shaft 114 may include one or more working lumens for receiving the fluid and/or other medical devices. In some embodiments, the distal tip 116 may include an elevator configured to manipulate a guidewire, a tool, a medical instrument, etc. extending through the elongate shaft 114. The handle 112 may include an elevator control 128 operably connected to the elevator. In some embodiments, the at least one motor may include a motor configured to control and/or power movement of the elevator. In some embodiments, an elevator motor may be disposed within the handle 112 of the endoscope 110.

In some embodiments, the location of the distal tip 116 and/or the distal end of the elongate shaft 114 may be tracked during use. For example, a mapping and navigation system may include an operating table (or other procedural or examination table or chair, etc.) configured to act or function as an electromagnetic generator to generate a magnetic field of a known geometry. Alternatively, or additionally, an electromagnetic generator separate from the operating table may be provided. The operating table and/or the electromagnetic generator may be coupled to a control unit which may include among other features, a processor, a memory, a display, and an input means. A position sensor (e.g., the electromagnetic sensor, etc.) or antenna, may be incorporated into the distal tip 116 and/or the distal end of the elongate shaft 114 of the endoscope 110. The position sensor may be configured for use in sensing a location of the position sensor in the magnetic field of the mapping and navigation system. In some embodiments, the position sensor may be electronically coupled to the workstation. When the position sensor is in the magnetic field, the location of the position sensor can be mathematically determined relative to the electromagnetic field source (e.g., the operating table and/or the electromagnetic generator). The workstation and the control unit may communicate to determine the position of the position sensor relative to the patient.

According to the invention, the motor control assembly 140 includes a first gear assembly 150 disposed within the motor control housing 142, as seen in FIGS. 3 and 4. The first gear assembly 150 includes a first worm gear 154 and a first worm gear shaft 152 configured to engage the first worm gear 154. In some embodiments, the first worm gear shaft 152 may be coupled to the first motor 144 by a first motor coupler 156. In some embodiments, the first worm gear shaft 152 may be coupled directly to the first motor 144. Other configurations are also contemplated. In some embodiments, the motor control assembly 140 may include a second gear assembly 160 disposed within the motor control housing 142, as seen in FIGS. 4 and 5. In some embodiments, the second gear assembly 160 may include a second worm gear 164 and a second worm gear shaft (not visible) configured to engage the second worm gear 164. In some embodiments, the second worm gear shaft may be coupled to the second motor 146 by a second motor coupler 166. In some embodiments, the second worm gear shaft may be coupled directly to the second motor 146. Other configurations are also contemplated. In some embodiments, the motor control assembly 140 may include the first gear assembly 150 and the second gear assembly 160 disposed within the motor control housing 142.

According to the invention, the endoscopic system 100 includes a first drive axle 170 extending from the first gear assembly 150 to the first deflection mechanism 120 disposed within the handle 112, as seen in FIGS. 4 and 5. The first drive axle 170 is configured to engage the first gear assembly 150 with the first deflection mechanism 120 disposed within the handle 112. In some embodiments, the first drive axle 170 may be configured to slidably engage the first gear assembly 150. In some embodiments, the first drive axle 170 may be fixedly secured to the first deflection mechanism 120.

In some embodiments, the endoscopic system 100 may include a second drive axle 180 extending from the first gear assembly 150 to the first deflection mechanism 120 disposed within the handle 112, as seen in FIGS. 4 and 5. In some embodiments, the second drive axle 180 may be configured to engage the second gear assembly 160 with the second deflection mechanism 122 disposed within the handle 112. In some embodiments, the second drive axle 180 may be configured to slidably engage the second gear assembly 160. In some embodiments, the second drive axle 180 may be fixedly secured to the second deflection mechanism 122.

In some embodiments, the first drive axle 170 may be oriented parallel with the second drive axle 180. In some embodiments, the first drive axle 170 may be coaxially disposed with respect to the second drive axle 180, as seen in FIG. 5. In some embodiments, a first portion of the first drive axle 170 may include a circular cross-sectional shape and a second portion of the first drive axle 170 may include a non-circular cross-sectional shape (e.g., polygonal, oblong, triangular, square, star shaped, hex shaped, etc.). The second portion of the first drive axle 170 may be configured to engage with the first worm gear 154. In some embodiments, the first drive axle 170 may be non-rotatable with respect to the first worm gear 154. Other configurations are also contemplated.

In some embodiments, a first portion of the second drive axle 180 may include a circular cross-sectional shape and a second portion of the second drive axle 180 may include a non-circular cross-sectional shape (e.g., polygonal, oblong, triangular, square, star shaped, hex shaped, etc.). The second portion of the second drive axle 180 may be configured to engage with the second worm gear 164. In some embodiments, the second drive axle 180 may be non-rotatable with respect to the second worm gear 164. Other configurations are also contemplated.

In some embodiments, the first portion of the first drive axle 170 may be disposed within the lumen of the second drive axle 180. Accordingly, in some embodiments, at least a portion of the first drive axle 170 may be coaxially disposed within the lumen of the second drive axle 180. The first drive axle 170 may be rotatable relative to the second drive axle 180. As such, the first drive axle 170 and the second drive axle 180 may be rotatable independently of each other. For example, in some embodiments, the first drive axle 170 may rotate and the second drive axle 180 may remain stationary. In some embodiments, the second drive axle 180 may rotate and the first drive axle 170 may remain stationary. In some embodiments, the first drive axle 170 may rotate in a first direction and the second drive axle 180 may rotate in a second direction, wherein the second direction may be the first direction, or the second direction may be different from the first direction. In some embodiments, the first drive axle 170 may rotate at a different speed from the second drive axle 180.

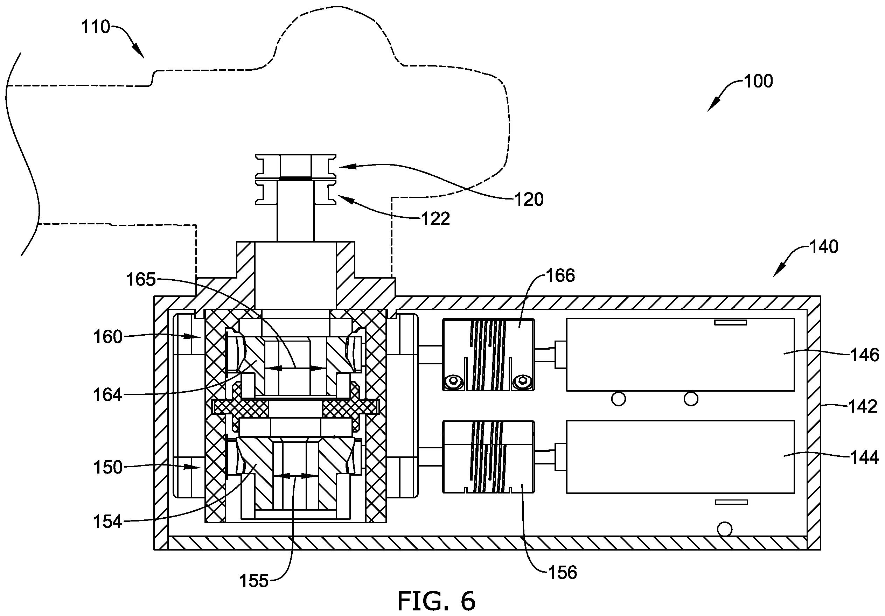

In some embodiments, the first drive axle 170 may define a first maximum outer extent and the second drive axle 180 may define a second maximum outer extent, wherein the second maximum outer extent is greater than the first maximum outer extent, as seen in FIG. 5. Accordingly, the first worm gear 154 may define a first maximum inner extent 155 and the second worm gear 164 may define a second maximum inner extent 165, wherein the second maximum inner extent 165 is greater than the first maximum inner extent 155, as seen in FIG. 6.

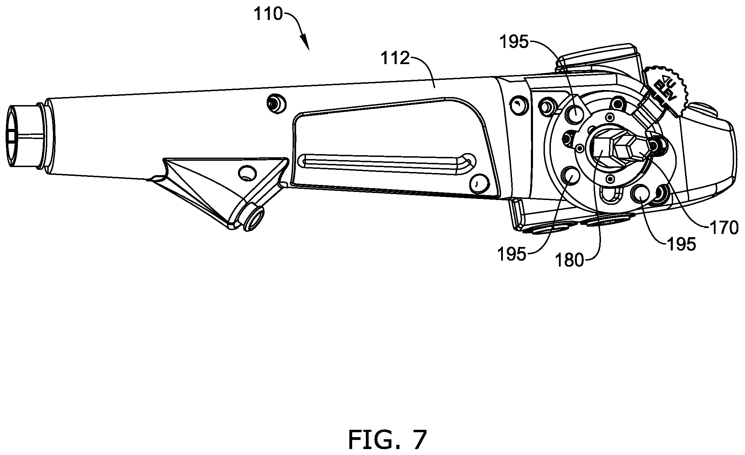

As discussed herein, in some embodiments, the first drive axle 170 may be fixedly attached and/or secured to the first deflection mechanism 120, and/or the second drive axle 180 may be fixedly attached and/or secured to the second deflection mechanism 122. As such, when the motor control assembly 140 and/or the motor control housing 142 (and the associated internal components - e.g., the first motor 144, the second motor 146, the first gear assembly 150, the second gear assembly 160, etc.) is detached from the handle 112 of the endoscope 110, as shown in FIG. 7, the first drive axle 170 and the second drive axle 180 may remain with and/or coupled to the handle 112 of the endoscope 110. In some embodiments, the first drive axle 170 and the second drive axle 180 may extend outward from a side of the handle 112 of the endoscope 110. Accordingly, the first drive axle 170 may be slidably received by the first gear assembly 150 and the second drive axle 180 may be slidably received by the second gear assembly 160 when the motor control assembly 140 and/or the motor control housing 142 is assembled to and/or releasably attached to the handle 112 of the endoscope 110. In some embodiments, the handle 112 of the endoscope 110 may include a plurality of spring-loaded plungers 195 configured to bias the motor control housing 142 away from the handle 112 of the endoscope 110 to remove any slack between the motor control housing 142 and the handle 112 of the endoscope 110.

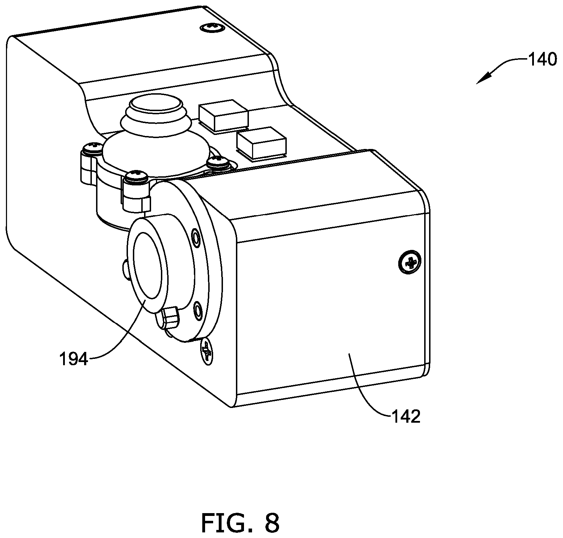

FIG. 8 illustrates the motor control assembly 140 and the motor control housing 142 detached from the handle 112 of the endoscope 110. In some embodiments, the motor control housing 142 may include a securement element 194 extending outward from the motor control housing 142. The securement element 194 may be configured to engage with and/or secure to the handle 112 of the endoscope 110. In some embodiments, the securement element 194 may include a first portion of a twist-lock mechanism and the handle 112 of the endoscope 110 may include a second portion of the twist-lock mechanism, wherein the second portion of the twist-lock mechanism is configured to engage with and/or receive the first portion of the twist-lock mechanism to detachably interface and/or secure the motor control assembly 140 and/or the motor control housing 142 to the handle 112 of the endoscope 110. In some embodiments, the first drive axle 170 and the second drive axle 180 (e.g., FIG. 7) may be configured to extend into and/or through the securement element 194 and/or the twist-lock mechanism. Other configurations and/or means of securing the motor control assembly 140 and/or the motor control housing 142 to the handle 112 of the endoscope 110 are also contemplated. For example, in some embodiments, the motor control housing 142 may be attached to the handle 112 using mechanical fasteners (e.g., screws, bolts, latches, cam locks, etc.). In some embodiments, the motor control housing 142 may be attached to the handle 112 using one or more magnets. In some alternative embodiments, the motor control housing 142 may include a plurality of spring-loaded plungers (not shown) configured to bias the motor control housing 142 away from the handle 112 of the endoscope 110 to remove any slack between the motor control housing 142 and the handle 112 of the endoscope 110.

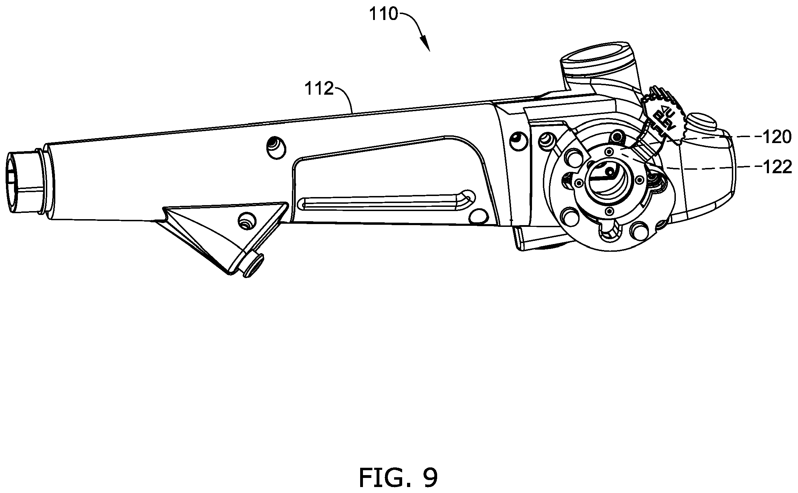

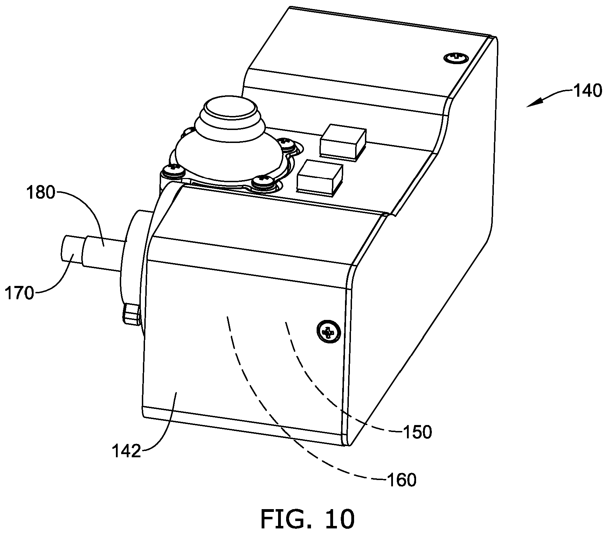

In an alternative configuration illustrated in FIGS. 9 and 10, the endoscopic system 100 may be configured as described above, except as specifically disclosed herein. In some embodiments, the first drive axle 170 may be configured to slidably engage the first deflection mechanism 120 and the second drive axle 180 may be configured to slidably engage the second deflection mechanism 122. In some embodiments, the first drive axle 170 may be fixedly attached and/or secured to the first gear assembly 150 and the second drive axle 180 may be fixedly attached and/or secured to the second gear assembly 160. In some embodiments, the first drive axle 170 may be releasably attached to the first gear assembly 150 and/or the second drive axle 180 may be releasably attached to the second gear assembly 160.

As such, when the motor control assembly 140 and/or the motor control housing 142 (and the associated internal components - e.g., the first motor 144, the second motor 146, the first gear assembly 150, the second gear assembly 160, etc.) is detached from the handle 112 of the endoscope 110, the first drive axle 170 and the second drive axle 180 may remain with and/or coupled to the motor control assembly 140, as seen in FIG. 10. In some embodiments, the first drive axle 170 and the second drive axle 180 may extend outward from a side of the motor control assembly 140 and/or the motor control housing 142. Accordingly, the first drive axle 170 may be slidably received by the first deflection mechanism 120 and the second drive axle 180 may be slidably received by the second deflection mechanism 122 when the motor control assembly 140 and/or the motor control housing 142 is assembled to and/or releasably attached to the handle 112 of the endoscope 110.

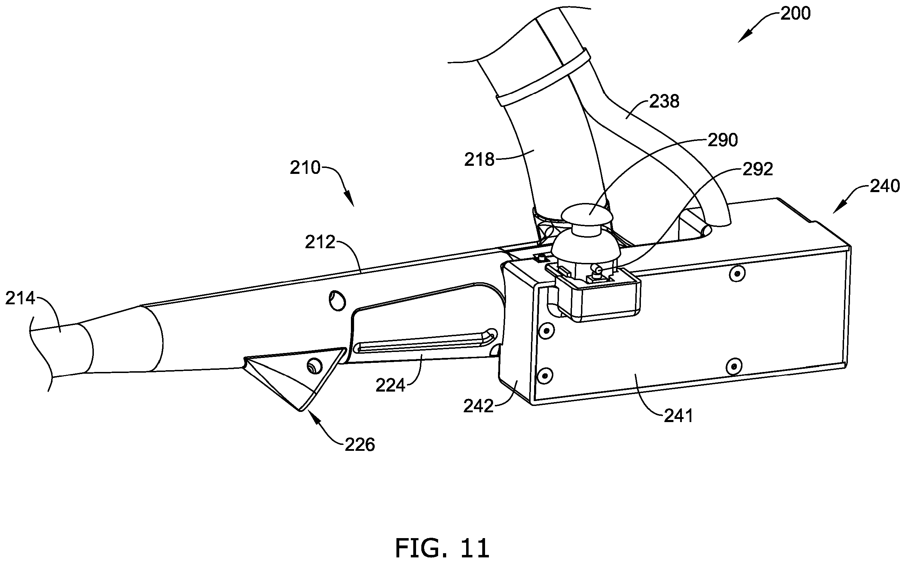

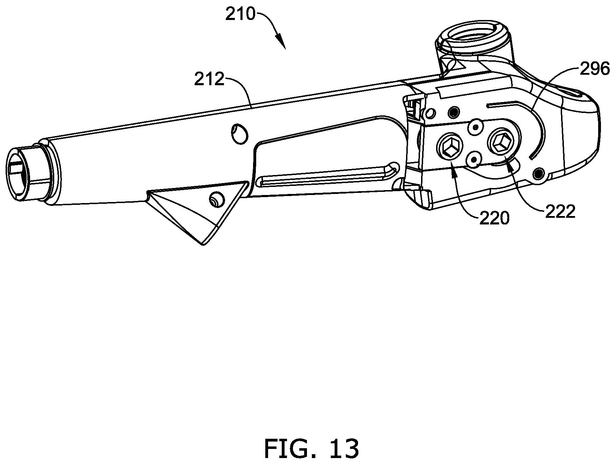

FIGS. 11-14 illustrate an alternative configuration of a medical device, depicted as an endoscopic system 200. In some embodiments, the endoscopic system 200 may include an endoscope 210. The endoscope 210 may be specific to a particular endoscopic procedure, such as, e.g., a ureteroscope, a cystoscope, a nephroscope, a duodenoscope, etc., or may be a general-purpose device suitable for a wide variety of procedures. In some embodiments, the endoscope 210 includes a handle 212 and an elongate shaft 214 extending distally from the handle 212 to a distal tip (not shown), wherein the distal tip may be similar in form and/or function to the distal tip 116 described herein. The handle 212 may include a first deflection mechanism 220 (e.g., FIG. 13) configured to deflect and/or articulate the distal tip of the elongate shaft 214 in a first plane and a second deflection mechanism 222 (e.g., FIG. 13) configured to deflect and/or articulate the distal tip of the elongate shaft 214 in a second plane different from the first plane, wherein the first deflection mechanism 220 may be similar in form and/or function to the first deflection mechanism 120 described herein and the second deflection mechanism 222 may be similar in form and/or function to the second deflection mechanism 122 described herein. In some embodiments, the first plane may be oriented at a non-zero angle to the second plane. In some embodiments, the first plane may be oriented perpendicular to the second plane. Other configurations are also contemplated. Although depicted as an endoscopic system 200 including an endoscope 210, it is noted and understood that features, components, and/or functionality described herein may be incorporated into another medical device, such as a steerable catheter or other medical device having one or more deflection mechanisms for controlling deflection and/or articulation of a distal tip of the elongate shaft of the medical device to facilitate navigation of the elongate shaft through the anatomy of a patient. Accordingly, the described components such as the handle, elongate shaft, deflection mechanisms, motor control assembly, and other components may be associated with another medical device having a deflectable/steerable distal tip of an elongate shaft, as desired.

In some embodiments, the first deflection mechanism 220 may include a first pulley or rotating member disposed within the handle 212 and operatively connected to the distal tip. In some embodiments, one or more first cables, wires, or other filaments may be engaged with and/or connected to the first pulley within the handle 212. In some embodiments, the one or more first cables, wires, or filaments may be engaged with and/or connected to the distal tip, such that tension applied to the one or more first cables, wires, or filaments by the first pulley deflects and/or articulates the distal tip in the first plane. In some embodiments, the second deflection mechanism 222 may include a second pulley or rotating member disposed within the handle 212 and operatively connected to the distal tip. In some embodiments, one or more second cables, wires, or other filaments may be engaged with and/or connected to the second pulley within the handle 212. In some embodiments, the one or more second cables, wires, or filaments may be engaged with and/or connected to the distal tip, such that tension applied to the one or more second cables, wires, or filaments by the second pulley deflects and/or articulates the distal tip in the second plane. Other configurations are also contemplated.

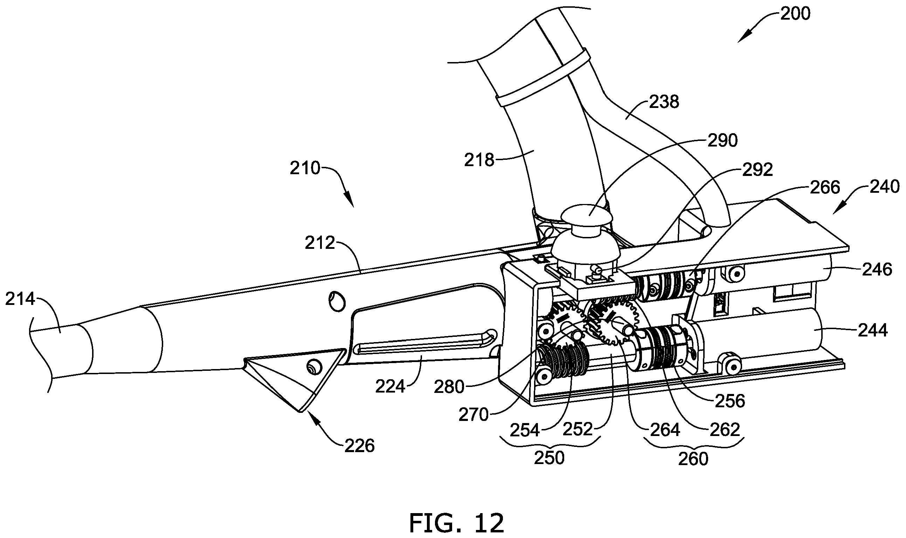

In some embodiments, the endoscopic system 200 may include a motor control assembly 240 including a motor control housing 242 configured to detachably interface with the handle 212 of the endoscope 210. In some embodiments, the motor control housing 242 may include a removable cover 241, as seen in FIG. 11, wherein internal components of the motor control assembly 240 may be accessed after removing the removable cover 241, as seen in FIG. 12. Additionally, in some embodiments, the motor control assembly 240 may include one or more internal covers disposed within the motor control housing 242 that may protect certain components and/or groups of components from each other, contamination, etc. In some embodiments, the one or more internal covers may provide structural support for selected internal components. It shall be understood that the presence and/or use of all, some, or any of the one or more internal covers is optional and is not required.

In some embodiments, the motor control assembly 240 may include at least one motor disposed within the motor control housing 242. In some embodiments, the at least one motor may include a first motor 244 disposed within the motor control housing 242. In some embodiments, the at least one motor may include a second motor 246 disposed within the motor control housing 242. In some embodiments, the at least one motor may include the first motor 244 disposed within the motor control housing 242 and the second motor 246 disposed within the motor control housing 242. In some embodiments, for each controlled degree of freedom of the distal tip, an individual motor may be added to the at least one motor.

The at least one motor may be electric and may be brushed or brushless DC motors. The at least one motor may provide the user partial force assistance for deflecting the distal tip, from 0% to 100% force assistance, thereby allowing for fully manual control, assisted control with reduced lever/knob force, or fully actuated control. Each motor may be attached to a rotary encoder which provides a relative position of said motor to the processor unit. In some embodiments, the at least one motor may be controller by a central processing unit (CPU), a microprocessor, and/or a combination thereof. In some embodiments, additional inputs for the CPU, the microprocessor, and/or the combination thereof may include components of the user interface, tactile feedback electronics, etc.

In some embodiments, the motor control assembly 240 may include a user input mechanism. In some embodiments, the user input mechanism may include one or more of a joystick control, a scroll wheel, a knob, a slider, a keypad, a touch screen interface or control, a voice interface or control, etc. In some embodiments, the motor control assembly 240 may include a joystick control 290 configured to operate the at least one motor disposed within the motor control housing 242. In some embodiments, the joystick control 290 may be configured to operate the first motor 244. In some embodiments, the joystick control 290 may be configured to operate the second motor 246. In some embodiments, the joystick control 290 may be configured to operate the first motor 244 and the second motor 246. In some embodiments, the joystick control 290 may be configured to operate the first motor 244 and the second motor 246 simultaneously. In some embodiments, the joystick control 290 may be configured to operate the first motor 244 and the second motor 246 independently of each other. Other configurations are also contemplated.

In some embodiments, the motor control assembly 240 may include a homing feature configured to return the distal tip to a home position (e.g., to a straightened and/or non-deflected configuration) and/or a speed control feature configured to control speed and/or responsiveness of movement of the distal tip. In some embodiments, the homing feature and/or the speed control feature may each include an input mechanism. For example, the input mechanism may be one or more buttons, a voice interface and/or control, a gesture interface and/or control, or other suitable means of providing input to the motor control assembly 240 and/or the user interface.

In some embodiments, the motor control assembly 240 may include one or more buttons 292. In some embodiments, the one or more buttons 292 may include a first button (e.g., a home button) configured to return the distal tip to a home position (e.g., to a straightened configuration), wherein activation of the first button automatically actuates the at least one motor to move the distal tip to the home position. In some embodiments, the one or more buttons 292 may include a second button that is user configurable to retain a saved position or configuration, wherein activation of the second button automatically actuates the at least one motor to move the distal tip to the saved position and/or configuration. Additional buttons and/or other configurations are also contemplated. Additionally, while the one or more buttons 292 are discussed herein by way of example, the motor control assembly 240 is not limited to the use of physical buttons and may include other input mechanisms.

In some embodiments, the motor control assembly 240 may include a self-homing feature and/or procedure. In one example, upon connecting the motor control assembly 240 to the handle 212, a homing program and/or algorithm is activated and/or run for the first motor 244 and the second motor 246 (where present) sequentially (e.g., the procedure is done on the first motor 244 and then is done on the second motor 246). In some instances, the homing program and/or algorithm is automatically initiated when the motor control assembly 240 is connected to the handle 212. In other instances, the user may push a button, or otherwise manually initiate the homing program and/or algorithm after the motor control assembly 240 is connected to the handle 212. The first motor 244 may start rotating in a first direction while the current is being monitored by the homing program and/or algorithm. Upon registering, encountering, and/or identifying a sudden increase in the amount of current drawn by the first motor 244, a location of an encoder is saved as a checkpoint for an upper limit or a lower limit. The first motor 244 then starts turning in a second direction opposite the first direction and again upon registering, encountering, and/or identifying a sudden increase in the amount of current drawn by the first motor 244, the value of the encoder is saved as a second checkpoint for the other limit of the motion range for that axis and/or the first motor 244. After determining the upper limit and the lower limit of the first motor 244 (and finding their corresponding values in terms of encoder values) the algorithm may divide the number of ticks between the upper limit and the lower limit by two. The corresponding encoder value would be the middle of the motion range or the home position for that axis and/or the first motor 244.

The same procedure is then repeated for the second motor 246, wherein the second motor 246 may start rotating in the first direction while the current is being monitored by the homing program and/or algorithm. Upon registering, encountering, and/or identifying a sudden increase in the amount of current drawn by the second motor 246, a location of an encoder is saved as a checkpoint for an upper limit or a lower limit. The second motor 246 then starts turning in the second direction opposite the first direction and again upon registering, encountering, and/or identifying a sudden increase in the amount of current drawn by the second motor 246, the value of the encoder is saved as a second checkpoint for the other limit of the motion range for that axis and/or the second motor 246. After determining the upper limit and the lower limit of the second motor 246 (and finding their corresponding values in terms of encoder values) the algorithm may divide the number of ticks between the upper limit and the lower limit by two. The corresponding encoder value would be the middle of the motion range or the home position for that axis and/or the second motor 246.

In another example, the homing feature and/or procedure may be manually activated after engaging the motor control assembly 240 to the handle 212 of the endoscope 210. Other configurations are also contemplated.

In some embodiments, the joystick control 290 may be configured to control movement of the distal tip. In some embodiments, the joystick control 290 may be configured to control a speed at which the distal tip moves. In some embodiments, the motor control assembly 240 may include a speed change control button. In some embodiments, the speed change control button may be built into and/or may be integrated into the joystick control 290. For example, in some embodiments, pressing axially on the joystick control 290 toward the motor control housing 242 may actuate the speed change control button. The speed change control button may actuate and/or cycle through a plurality of speed settings to permit at least some degree of customization over movement of the distal tip. In some embodiments, the plurality of speed settings may include at least a high-speed setting which permits faster control and/or faster movement of the distal tip and a low speed setting which permits finer control and/or slower movement of the distal tip. In some embodiments, the plurality of speed settings may further include a medium speed setting and/or other speed settings (e.g., medium-high, medium low, extra low, etc.).

In some embodiments, the motor control housing 242, the speed change control feature, the speed change control button, and/or the plurality of speed settings may be user customizable. In some embodiments, the user may be able to set speed mode(s), select and/or set desired speed(s), and/or set or save the speed mode(s) and/or desired speed(s). In some embodiments, the speed change control button may be depressed and held to set or save the speed mode(s) and/or desired speed(s). In some embodiments, the motor control housing 242 and/or the user interface may include additional buttons, a keypad, a scroll wheel, a dial, a touch interface, or other input mechanism for setting and/or adjusting the speed mode(s) and/or desired speed(s). These are only examples, and other configurations are also contemplated.

In some embodiments, other control mechanisms may be used in place of the joystick control 290. In some embodiments, the motor control assembly 240 may include a keypad. In some embodiments, the motor control assembly 240 may include a scroll wheel. In some embodiments, the motor control assembly 240 may include a touch screen. Other control configurations are also contemplated.

In some embodiments, the distal tip may include a camera and may, for example, have deflection and/or articulation capabilities in one or more directions for viewing patient anatomy. **In** some embodiments, the endoscope 210 may be a duodenoscope such as an Exalt^{™} Model D scope. However, other medical devices, such as another endoscope (e.g., a ureteroscope, etc.) or related system, (e.g., Lithovue^{™}, SpyScope^{™} DS, SpyGlass^{™} DS, etc.) may be used in addition to or in place of a duodenoscope and/or the endoscope 210. In some embodiments, the endoscope 210 may be configured to deliver fluid from a fluid management system to a treatment site via the elongate shaft 214. The elongate shaft 214 may include one or more working lumens for receiving a flow of fluid and/or other medical devices therethrough. In some embodiments, the endoscope 210 may be connected to the fluid management system via one or more supply line(s).

In some embodiments, the handle 212 of the endoscope 210 may include a plurality of elements configured to facilitate the endoscopic procedure. In some embodiments, an umbilicus 218 extends from the handle 212 and is configured for attachment to an electronic device (not pictured) such as e.g. a computer system, a console, a microcontroller, etc. for providing power, analyzing endoscopic data, controlling the endoscopic intervention, and/or performing other functions. In some embodiments, the electronic device to which the umbilicus 218 is connected may have functionality for recognizing and exchanging data with other endoscopic accessories. The handle 212 may include a grip area 224 for the operating physician to grasp while performing the endoscopic procedure. In some embodiments, the handle 212 may include a side port 226 in communication with the one or more working lumens of the elongate shaft 214 and/or the endoscope 210.

In some embodiments, a motor control umbilicus 238 may extend from the motor control housing 242 and may be configured for attachment to an electronic device, which may be the same electronic device that the umbilicus 218 is attached to or may be a different electronic device as desired, for providing power, controlling endoscopic intervention and/or the motor control assembly, and/or other functions. In some embodiments, the motor control umbilicus 238 may be secured to the umbilicus 218. In some embodiments, the motor control umbilicus 238 may be releasably and/or removably secured to the umbilicus 218. In some embodiments, the motor control umbilicus 238 may be secured to the umbilicus 218 with a hook and loop closure device (e.g., Velcro^{™}), tape, a wire tie, or other securement apparatus.

In some embodiments, the endoscope 210 may be in electronic communication with a workstation via a wired connection (e.g., the umbilicus 218). In some embodiments, the workstation may include a touch panel computer, an interface box for receiving the wired connection (e.g., the umbilicus 218), a cart, and a power supply, among other features. In some embodiments, the interface box may be configured with a wired or wireless communication connection with the controller of the fluid management system. The touch panel computer may include at least a display screen and an image processor. In some embodiments, the workstation may be a multi-use component (e.g., used for more than one procedure) while the endoscope 210 may be a single use device, although this is not required. In some embodiments, the workstation may be omitted and the endoscope 210 may be electronically coupled directly to the controller of a fluid management system, where used.

In some embodiments, the handle 212 may include at least one communication interface for attaching accessory devices. In some embodiments, the handle 212 may include Universal Serial Bus type-C (USB-C) ports, Universal Serial Bus (USB) ports, ethernet ports, and/or other types of ports. In some embodiments, more, less, and/or other communication interfaces of various types, including, for example, custom interfaces, may be used. In some embodiments, the handle 212 has only one communication interface but may be connectable to e.g. a USB hub with multiple ports for connecting multiple accessories. In some embodiments, the at least one communication interface may provide power to the accessory device(s) in addition to exchanging data therewith. Thus, the accessory device(s) need not have separate cables running to a connected electronic device or a battery that adds additional weight to the handle 212. In some embodiments, the accessory device(s) may be uniquely associated with the endoscope 210 and recognized by the electronic device through "plug and play" functionality without any user setup required.

In some embodiments, the endoscope 210 may include one or more sensors proximate the distal tip and/or the distal end of the elongate shaft 214. For example, the endoscope 210 may include a pressure sensor at the distal tip of the elongate shaft 214 to measure intracavity pressure within the treatment site. The endoscope 210 may also include other sensors such as, for example, a temperature sensor, a Fiber Bragg grating optical fiber to detect stresses, and/or an antenna or electromagnetic sensor (e.g., a position sensor). In some embodiments, the distal tip and/or the distal end of the endoscope 210 may also include at least one camera to provide a visual feed to the user on the display screen of the touch panel computer. In another embodiment, the endoscope 210 may include two cameras having different communications requirements or protocols so that different information may be relayed to the user by each camera. When so provided, the user may switch back and forth between the cameras at will through the touch screen interface and/or the touch panel computer. While not explicitly shown, the elongate shaft 214 may include one or more working lumens for receiving the fluid and/or other medical devices. In some embodiments, the distal tip may include an elevator configured to manipulate a guidewire, a tool, a medical instrument, etc. extending through the elongate shaft 214, and the handle 212 may include an elevator control operably connected to the elevator. In some embodiments, the at least one motor may include a motor configured to control and/or power movement of the elevator. In some embodiments, an elevator motor may be disposed within the handle 212 of the endoscope 210.

In some embodiments, the location of the distal tip and/or the distal end of the elongate shaft 214 may be tracked during use. For example, a mapping and navigation system may include an operating table (or other procedural or examination table or chair, etc.) configured to act or function as an electromagnetic generator to generate a magnetic field of a known geometry. Alternatively, or additionally, an electromagnetic generator separate from the operating table may be provided. The operating table and/or the electromagnetic generator may be coupled to a control unit which may include among other features, a processor, a memory, a display, and an input means. A position sensor (e.g., the electromagnetic sensor, etc.) or antenna, may be incorporated into the distal tip and/or the distal end of the elongate shaft 214 of the endoscope 210. The position sensor may be configured for use in sensing a location of the position sensor in the magnetic field of the mapping and navigation system. In some embodiments, the position sensor may be electronically coupled to the workstation. When the position sensor is in the magnetic field, the location of the position sensor can be mathematically determined relative to the electromagnetic field source (e.g., the operating table and/or the electromagnetic generator). The workstation and the control unit may communicate to determine the position of the position sensor relative to the patient.

In some embodiments, the motor control assembly 240 may include a first gear assembly 250 disposed within the motor control housing 242. In some embodiments, the first gear assembly 250 may include a first worm gear 254 and a first worm gear shaft 252 configured to engage the first worm gear 254. In some embodiments, the first worm gear shaft 252 may be coupled to the first motor 244 by a first motor coupler 256. In some embodiments, the first worm gear shaft 252 may be coupled directly to the first motor 244. Other configurations are also contemplated. In some embodiments, the motor control assembly 240 may include a second gear assembly 260 disposed within the motor control housing 242. In some embodiments, the second gear assembly 260 may include a second worm gear 264 and a second worm gear shaft 262 configured to engage the second worm gear 264. In some embodiments, the second worm gear shaft 262 may be coupled to the second motor 246 by a second motor coupler 266. In some embodiments, the second worm gear shaft 262 may be coupled directly to the second motor 246. Other configurations are also contemplated. In some embodiments, the motor control assembly 240 may include the first gear assembly 250 and the second gear assembly 260 disposed within the motor control housing 242.

In some embodiments, the endoscopic system 200 may include a first drive axle 270 extending from the first gear assembly 250 to the first deflection mechanism 220 disposed within the handle 212. In some embodiments, the first drive axle 270 may be configured to engage the first gear assembly 250 with the first deflection mechanism 220 disposed within the handle 212. In some embodiments, the first drive axle 270 may be configured to slidably engage the first deflection mechanism 220. In some embodiments, the first drive axle 270 may be fixedly secured to the first gear assembly 250 and/or the first worm gear 254. Other configurations are also contemplated.

In some embodiments, the endoscopic system 200 may include a second drive axle 280 extending from the first gear assembly 250 to the first deflection mechanism 220 disposed within the handle 212. In some embodiments, the second drive axle 280 may be configured to engage the second gear assembly 260 with the second deflection mechanism 222 disposed within the handle 212. In some embodiments, the second drive axle 280 may be configured to slidably engage the second deflection mechanism 222. In some embodiments, the second drive axle 280 may be fixedly secured to the second gear assembly 260 and/or the second worm gear 264. Other configurations are also contemplated.

In some embodiments, the first drive axle 270 may be oriented parallel with the second drive axle 280. In some embodiments, the first drive axle 270 may be laterally offset from and/or with respect to the second drive axle 280. In some embodiments, a first portion of the first drive axle 270 may include a circular cross-sectional shape and a second portion of the first drive axle 270 may include a non-circular cross-sectional shape (e.g., polygonal, oblong, triangular, square, star shaped, hex shaped, etc.). The second portion of the first drive axle 270 may be configured to slidably and/or releasably engage with the first deflection mechanism 220. In some embodiments, the first drive axle 270 may be non-rotatable with respect to and/or relative to the first worm gear 254. In some embodiments, the first portion of the first drive axle 270 may include a non-circular cross-sectional shape. In some embodiments, the first portion of the first drive axle 270 may include the same cross-sectional shape as the second portion of the first drive axle 270. Other configurations are also contemplated.

In some embodiments, a first portion of the second drive axle 280 may include a circular cross-sectional shape and a second portion of the second drive axle 280 may include a non-circular cross-sectional shape (e.g., polygonal, oblong, triangular, square, star shaped, hex shaped, etc.). The second portion of the second drive axle 280 may be configured to slidably and/or releasably engage with the second deflection mechanism 222. In some embodiments, the second drive axle 280 may be non-rotatable with respect to and/or relative to the second worm gear 264. In some embodiments, the first portion of the second drive axle 280 may include a non-circular cross-sectional shape. In some embodiments, the first portion of the second drive axle 280 may include the same cross-sectional shape as the second portion of the second drive axle 280. Other configurations are also contemplated.

In some embodiments, the first drive axle 270 and the second drive axle 280 may be rotatable independently of each other. For example, in some embodiments, the first drive axle 270 may rotate and the second drive axle 280 may remain stationary. In some embodiments, the second drive axle 280 may rotate and the first drive axle 270 may remain stationary. In some embodiments, the first drive axle 270 may rotate in a first direction and the second drive axle 280 may rotate in a second direction, wherein the second direction may be the first direction, or the second direction may be different from the first direction. In some embodiments, the first drive axle 270 may rotate at a different speed from the second drive axle 280.

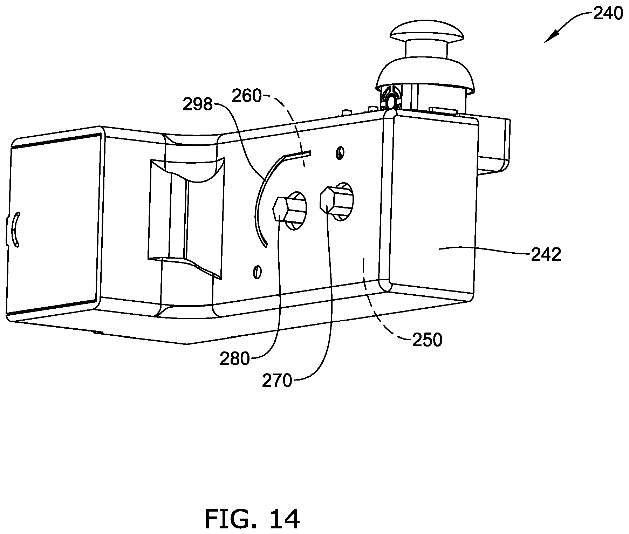

As discussed herein, in some embodiments, the first drive axle 270 may be fixedly attached and/or secured to the first gear assembly 250 and/or the first worm gear 254, and/or the second drive axle 280 may be fixedly attached and/or secured to the second gear assembly 260 and/or the second worm gear 264. As such, when the motor control assembly 240 and/or the motor control housing 242 (and the associated internal components - e.g., the first motor 244, the second motor 246, the first gear assembly 250, the second gear assembly 260, etc.) is detached from the handle 212 of the endoscope 210, as shown in FIGS. 13-14, the first drive axle 270 and the second drive axle 280 may remain with and/or coupled to the motor control assembly 240 and/or the motor control housing 242 (e.g., FIG. 14). In some embodiments, the first drive axle 270 and the second drive axle 280 may extend outward from a side of the motor control assembly 240 and/or the motor control housing 242. Accordingly, the first drive axle 270 may be slidably received by the first deflection mechanism 220 and the second drive axle 280 may be slidably received by the second deflection mechanism 222 when the motor control assembly 240 and/or the motor control housing 242 is assembled to and/or releasably attached to the handle 212 of the endoscope 210.

In some embodiments, the motor control housing 242 may include a securement element attached to, integrated with, and/or extending outward from the motor control housing 242. The securement element may be configured to engage with and/or secure to the handle 212 of the endoscope 210. In some embodiments, the securement element may include a first portion of a twist-lock mechanism and the handle 212 of the endoscope 210 may include a second portion of the twist-lock mechanism, wherein the second portion of the twist-lock mechanism is configured to engage with and/or receive the first portion of the twist-lock mechanism to detachably interface and/or secure the motor control assembly 240 and/or the motor control housing 242 to the handle 212 of the endoscope 210. In some embodiments, the first drive axle 270 and the second drive axle 280 may be configured to extend into and/or through the securement element and/or the twist-lock mechanism. Other configurations and/or means of securing the motor control assembly 240 and/or the motor control housing 242 to the handle 212 of the endoscope 210 are also contemplated. For example, in some embodiments, the motor control housing 242 may be attached to the handle 212 using mechanical fasteners (e.g., screws, bolts, latches, cam locks, etc.). In some embodiments, the motor control housing 242 may be attached to the handle 212 using one or more magnets. In some embodiments, the handle 212 of the endoscope 210 may include a plurality of spring-loaded plungers (not shown) configured to bias the motor control housing 242 away from the handle 212 of the endoscope 210 to remove any slack between the motor control housing 242 and the handle 212 of the endoscope 210. In some alternative embodiments, the motor control housing 242 may include a plurality of spring-loaded plungers (not shown) configured to bias the motor control housing 242 away from the handle 212 of the endoscope 210 to remove any slack between the motor control housing 242 and the handle 212 of the endoscope 210.

In some embodiments, the handle 212 of the endoscope 210 may include a first alignment feature 296 (e.g., FIG. 13) and the motor control housing 242 may include a second alignment feature 298 (e.g., FIG. 14) configured to engage with the first alignment feature when the motor control assembly 240 and/or the motor control housing 242 is detachably engaged with the handle 212 of the endoscope 210. In some embodiments, the first alignment feature may cooperate with the second alignment feature to prevent relative rotation between the handle 212 and the motor control housing 242.

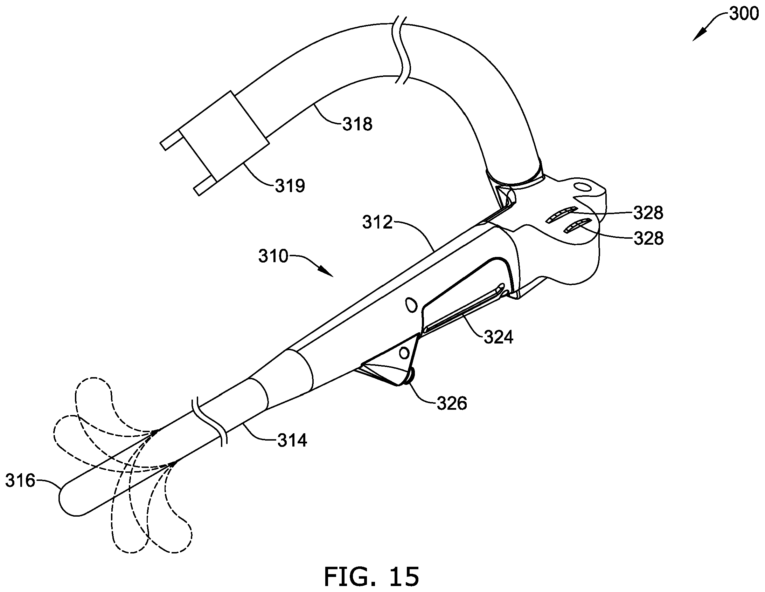

FIGS. 15-17 illustrates aspects of another alternative embodiment of a medical device, depicted as an endoscopic system 300. In some embodiments, the endoscopic system 300 may include an endoscope 310. The endoscope 310 may be specific to a particular endoscopic procedure, such as, e.g., a ureteroscope, a cystoscope, a nephroscope, a duodenoscope, etc., or may be a general-purpose device suitable for a wide variety of procedures. In some embodiments, the endoscope 310 includes a handle 312 and an elongate shaft 314 extending distally from the handle 312 to a distal tip 316, and an umbilicus 318 extending from the handle 312 to a proximal connector 319, as seen in FIG. 15. Although depicted as an endoscopic system 300 including an endoscope 310, it is noted and understood that features, components, and/or functionality described herein may be incorporated into another medical device, such as a steerable catheter or other medical device having one or more deflection mechanisms for controlling deflection and/or articulation of a distal tip of the elongate shaft of the medical device to facilitate navigation of the elongate shaft through the anatomy of a patient. Accordingly, the described components such as the handle, elongate shaft, deflection mechanisms, motor control assembly, controller and other components may be associated with another medical device having a deflectable/steerable distal tip of an elongate shaft, as desired.

In some embodiments, the distal tip 316 may include a camera and may, for example, have deflection and/or articulation capabilities in one or more directions for viewing patient anatomy. In some embodiments, the endoscope 310 may be a duodenoscope such as an Exalt^{™} Model D scope. However, other medical devices, such as another endoscope (e.g., a ureteroscope, etc.) or related system, (e.g., Lithovue^{™}, SpyScope^{™} DS, SpyGlass^{™} DS, etc.) may be used in addition to or in place of a duodenoscope and/or the endoscope 310. In some embodiments, the endoscope 310 may be configured to deliver fluid from a fluid management system to a treatment site via the elongate shaft 314. The elongate shaft 314 may include one or more working lumens for receiving a flow of fluid and/or other medical devices therethrough. In some embodiments, the endoscope 310 may be connected to the fluid management system via one or more supply line(s).

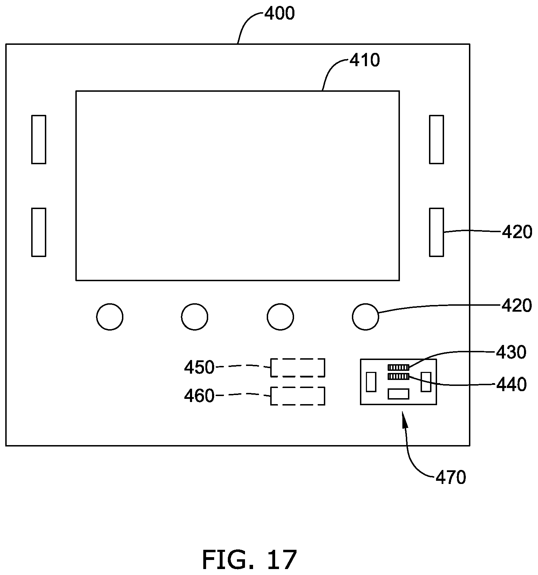

In some embodiments, the handle 312 of the endoscope 310 may include a plurality of elements configured to facilitate the endoscopic procedure. In some embodiments, the proximal connector 319 is configured to releasably couple to a controller 400 (e.g., FIG. 17) such as e.g. a computer system, a console, a microcontroller, etc. for providing power, analyzing endoscopic data, controlling the endoscopic intervention, and/or performing other functions. In some embodiments, the controller 400 to which the umbilicus 318 and/or the proximal connector 319 is releasably coupled may have functionality for recognizing and exchanging data with other endoscopic accessories. The handle 312 may include a grip area 324 for the operating physician to grasp while performing the endoscopic procedure. In some embodiments, the handle 312 may include a side port 326 in communication with the one or more working lumens of the elongate shaft 314 and/or the endoscope 310.

In some embodiments, the handle 312 may include at least one communication interface for attaching accessory devices. In some embodiments, the handle 312 may include Universal Serial Bus type-C (USB-C) ports, Universal Serial Bus (USB) ports, ethernet ports, and/or other types of ports. In some embodiments, more, less, and/or other communication interfaces of various types, including, for example, custom interfaces, may be used. In some embodiments, the handle 312 has only one communication interface but may be connectable to e.g. a USB hub with multiple ports for connecting multiple accessories. In some embodiments, the at least one communication interface may provide power to the accessory device(s) in addition to exchanging data therewith. Thus, the accessory device(s) need not have separate cables running to a connected electronic device or a battery that adds additional weight to the handle 312. In some embodiments, the accessory device(s) may be uniquely associated with the endoscope 310 and recognized by the electronic device through "plug and play" functionality without any user setup required.

In some embodiments, the endoscope 310 may include one or more sensors proximate the distal tip 316 and/or the distal end of the elongate shaft 314. For example, the endoscope 310 may include a pressure sensor at the distal tip 316 of the elongate shaft 314 to measure intracavity pressure within the treatment site. The endoscope 310 may also include other sensors such as, for example, a temperature sensor, a Fiber Bragg grating optical fiber to detect stresses, and/or an antenna or electromagnetic sensor (e.g., a position sensor). In some embodiments, the distal tip 316 and/or the distal end of the endoscope 310 may also include at least one camera to provide a visual feed to the user on the display screen of the touch panel computer. In another embodiment, the endoscope 310 may include two cameras having different communications requirements or protocols so that different information may be relayed to the user by each camera. When so provided, the user may switch back and forth between the cameras at will through the touch screen interface and/or the touch panel computer. While not explicitly shown, the elongate shaft 314 may include one or more working lumens for receiving the fluid and/or other medical devices. In some embodiments, the distal tip 316 may include an elevator configured to manipulate a guidewire, a tool, a medical instrument, etc. extending through the elongate shaft 314. The handle 312 may include an elevator control operably connected to the elevator. In some embodiments, the at least one motor may include a motor configured to control and/or power movement of the elevator. In some embodiments, an elevator motor may be disposed within the handle 312 of the endoscope 310.

In some embodiments, the location of the distal tip 316 and/or the distal end of the elongate shaft 314 may be tracked during use. For example, a mapping and navigation system may include an operating table (or other procedural or examination table or chair, etc.) configured to act or function as an electromagnetic generator to generate a magnetic field of a known geometry. Alternatively, or additionally, an electromagnetic generator separate from the operating table may be provided. The operating table and/or the electromagnetic generator may be coupled to a control unit which may include among other features, a processor, a memory, a display, and an input means. A position sensor (e.g., the electromagnetic sensor, etc.) or antenna, may be incorporated into the distal tip 316 and/or the distal end of the elongate shaft 314 of the endoscope 310. The position sensor may be configured for use in sensing a location of the position sensor in the magnetic field of the mapping and navigation system. In some embodiments, the position sensor may be electronically coupled to the workstation. When the position sensor is in the magnetic field, the location of the position sensor can be mathematically determined relative to the electromagnetic field source (e.g., the operating table and/or the electromagnetic generator). The workstation and the control unit may communicate to determine the position of the position sensor relative to the patient.

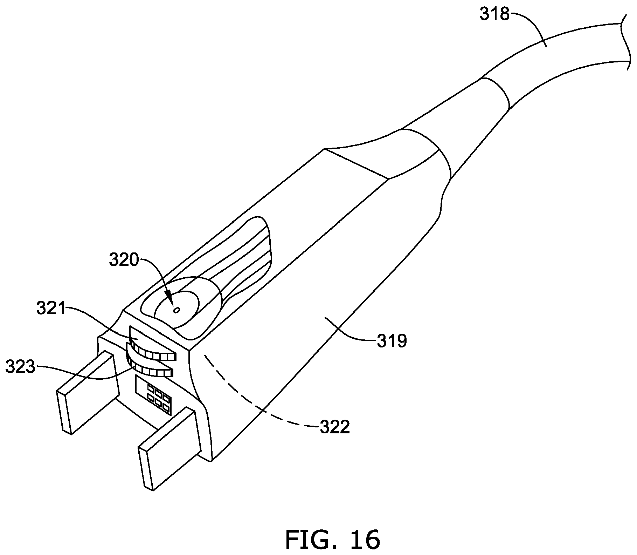

The proximal connector 319, shown in more detail in FIG. 16, may include a first deflection mechanism 320 disposed therein, the first deflection mechanism 320 being configured to deflect and/or articulate the distal tip 316 of the elongate shaft 314 in a first plane. In some embodiments, the proximal connector 319 may include a second deflection mechanism 322 disposed therein, the second deflection mechanism 322 being configured to deflect and/or articulate the distal tip 316 of the elongate shaft 314 in a second plane different from the first plane. In some embodiments, the first plane may be oriented at a non-zero angle to the second plane. In some embodiments, the first plane may be oriented perpendicular to the second plane. Other configurations are also contemplated.

In some embodiments, the first deflection mechanism 320 may include a first pulley or rotating member disposed within the proximal connector 319 and operatively connected to the distal tip 316. In some embodiments, one or more first cables, wires, or other filaments may be engaged with and/or connected to the first pulley within the proximal connector 319. In some embodiments, the one or more first cables, wires, or filaments may be engaged with and/or connected to the distal tip 316, such that tension applied to the one or more first cables, wires, or filaments by the first pulley deflects and/or articulates the distal tip 316 in the first plane. In some embodiments, the first deflection mechanism 320 may include a first gear 321 operably connected to the first deflection mechanism 320 and/or at least partially disposed within the proximal connector 319. In some embodiments, the first gear 321 may extend at least partially outside of the proximal connector 319. Other configurations are also contemplated.

In some embodiments, the second deflection mechanism 322 may include a second pulley or rotating member disposed within the proximal connector 319 and operatively connected to the distal tip 316. In some embodiments, one or more second cables, wires, or other filaments may be engaged with and/or connected to the second pulley within the proximal connector 319. In some embodiments, the one or more second cables, wires, or filaments may be engaged with and/or connected to the distal tip 316, such that tension applied to the one or more second cables, wires, or filaments by the second pulley deflects and/or articulates the distal tip 316 in the second plane. In some embodiments, the second deflection mechanism 322 may include a second gear 323 operably connected to the second deflection mechanism 322 and/or at least partially disposed within the proximal connector 319. In some embodiments, the second gear 323 may extend at least partially outside of the proximal connector 319. Other configurations are also contemplated.

In some embodiments, the endoscopic system may include a controller 400 (e.g., FIG. 17). The controller 400 may include a controller housing. In some embodiments, the controller 400 may include a touch panel computer 410, an interface box and/or an interface connector 470 for receiving a wired connection (e.g., the proximal connector 319 of the umbilicus 318), and a power supply, among other features. In some embodiments, the controller 400 may optionally include a cart or other means of rendering the controller 400 portable. The touch panel computer 410 may include at least a display screen and an image processor. In some embodiments, the controller 400 may be a multi-use component (e.g., used for more than one procedure) while the endoscope 310 may be a single use device, although this is not required. The controller 400 may include one or more buttons 420, which may be buttons, knobs, sliders, dials, scroll wheels, etc. for activating selected features, setting selected parameters, etc.

The interface connector 470 may be configured to receive and/or releasably couple to the proximal connector 319 of the endoscope 310. The interface connector 470 may be configured to transfer electrical power, mechanical power, mechanical torque, sensor data, camera data, etc. between the controller 400 and the endoscope 310. The controller 400 may include a first motor 450 disposed within the controller housing. The first motor 450 may be operably connected to a first drive gear 430 disposed proximate the interface connector 470. In some embodiments, the first drive gear 430 may extend at least partially into the interface connector 470 and/or may extend at least partially outside of the controller housing. In some embodiments, the first drive gear 430 may be configured to engage with and/or transfer mechanical energy and/or torque to the first gear 321 when the proximal connector 319 is releasably coupled to the controller 400 and/or the interface connector 470. As such, the first motor 450 may be configured to operatively engage the first deflection mechanism 320 (e.g., via the first drive gear 430 and the first gear 321) when the proximal connector 319 is releasably coupled to the controller 400 and/or the interface connector 470.

In some embodiments, the controller 400 may include a second motor 460 disposed within the controller housing. The second motor 460 may be operably connected to a second drive gear 440 disposed proximate the interface connector 470. In some embodiments, the second drive gear 440 may extend at least partially into the interface connector 470 and/or may extend at least partially outside of the controller housing. In some embodiments, the second drive gear 440 may be configured to engage with and/or transfer mechanical energy and/or torque to the second gear 323 when the proximal connector 319 is releasably coupled to the controller 400 and/or the interface connector 470. As such, the second motor 460 may be configured to operatively engage the second deflection mechanism 322 (e.g., via the second drive gear 440 and the second gear 323) when the proximal connector 319 is releasably coupled to the controller 400 and/or the interface connector 470.

The first motor 450 and/or the second motor 460 may be electric and may be brushed or brushless DC motors. The first motor 450 and/or the second motor 460 may provide the user partial force assistance for deflecting the distal tip 116, from 0% to 100% force assistance, thereby allowing for fully manual control, assisted control with reduced lever/knob force, or fully actuated control. Each motor may be attached to a rotary encoder which provides a relative position of said motor to the processor unit. In some embodiments, the first motor 450 and/or the second motor 460 may be controller by a central processing unit (CPU), a microprocessor, and/or a combination thereof. In some embodiments, additional inputs for the CPU, the microprocessor, and/or the combination thereof may include components of the user interface, tactile feedback electronics, etc.

Returning briefly to FIG. 15, in some embodiments, the handle 312 of the endoscope 310 may include at least one input mechanism 328 configured to control deflection of the distal tip 316 in the first plane and/or the second plane. In some embodiments, the at least one input mechanism 328 may be in electronic communication with the controller 400 when the proximal connector 319 is releasably coupled to the controller 400. In some embodiments, the at least one input mechanism 328 may be configured to operate the first motor 450. In some embodiments, the at least one input mechanism 328 may be configured to operate the second motor 460. In some embodiments, the at least one input mechanism 328 may be configured to operate the first motor 450 and the second motor 460. In some embodiments, the at least one input mechanism 328 may be configured to operate the first motor 450 and the second motor 460 simultaneously. In some embodiments, the at least one input mechanism 328 may be configured to operate the first motor 450 and the second motor 460 independently of each other. Other configurations are also contemplated.

In some embodiments, the controller 400 may include a homing feature configured to return the distal tip 316 to a home position (e.g., to a straightened configuration) and/or a speed control feature configured to control speed and/or responsiveness of movement of the distal tip 316. In some embodiments, the homing feature and/or the speed control feature may each include an input mechanism. For example, the input mechanism may be one or more buttons, a voice interface and/or control, a gesture interface and/or control, or other suitable means of providing input to the controller 400 and/or the user interface.

In some embodiments, the handle 312 of the endoscope 310 may include one or more buttons (not shown). In some embodiments, the one or more buttons may include a first button (e.g., a home button) configured to return the distal tip 316 to a home position (e.g., to a straightened configuration), wherein activation of the first button automatically actuates the first motor 450 and/or the second motor 460 to move the distal tip 316 to the home position. In some embodiments, the one or more buttons may include a second button that is user configurable to retain a saved position or configuration, wherein activation of the second button automatically actuates the first motor 450 and/or the second motor 460 to move the distal tip 316 to the saved position and/or configuration. Additional buttons and/or other configurations are also contemplated. Additionally, while the one or more buttons are discussed herein by way of example, the handle 312 of the endoscope 310 is not limited to the use of physical buttons and may include other input mechanisms.

In some embodiments, the controller 400 may include a self-homing feature and/or procedure. In one example, upon connecting the proximal connector 319 to the controller 400, a homing program and/or algorithm is activated and/or run for the first motor 450 and the second motor 460 sequentially (e.g., the procedure is done on the first motor 450 and then is done on the second motor 460). In some instances, the homing program and/or algorithm is automatically initiated when the proximal connector 319 is connected to the controller 400. In other instances, the user may push a button, or otherwise manually initiate the homing program and/or algorithm after the proximal connector 319 is connected to the controller 400. The first motor 450 may start rotating in a first direction while the current is being monitored by the homing program and/or algorithm. Upon registering, encountering, and/or identifying a sudden increase in the amount of current drawn by the first motor 450, a location of an encoder is saved as a checkpoint for an upper limit or a lower limit. The first motor 450 then starts turning in a second direction opposite the first direction and again upon registering, encountering, and/or identifying a sudden increase in the amount of current drawn by the first motor 450, the value of the encoder is saved as a second checkpoint for the other limit of the motion range for that axis and/or the first motor 450. After determining the upper limit and the lower limit of the first motor 450 (and finding their corresponding values in terms of encoder values) the algorithm may divide the number of ticks between the upper limit and the lower limit by two. The corresponding encoder value would be the middle of the motion range or the home position for that axis and/or the first motor 450.

The same procedure is then repeated for the second motor 460, wherein the second motor 460 may start rotating in the first direction while the current is being monitored by the homing program and/or algorithm. Upon registering, encountering, and/or identifying a sudden increase in the amount of current drawn by the second motor 460, a location of an encoder is saved as a checkpoint for an upper limit or a lower limit. The second motor 460 then starts turning in the second direction opposite the first direction and again upon registering, encountering, and/or identifying a sudden increase in the amount of current drawn by the second motor 460, the value of the encoder is saved as a second checkpoint for the other limit of the motion range for that axis and/or the second motor 460. After determining the upper limit and the lower limit of the second motor 460 (and finding their corresponding values in terms of encoder values) the algorithm may divide the number of ticks between the upper limit and the lower limit by two. The corresponding encoder value would be the middle of the motion range or the home position for that axis and/or the second motor 460.

In another example, the homing feature and/or procedure may be manually activated after engaging the proximal connector 319 to the controller 400. Other configurations are also contemplated.

In some embodiments, the at least one input mechanism 328 may be configured to control movement of the distal tip 316. In some embodiments, the at least one input mechanism 328 may be configured to control a speed at which the distal tip 316 moves. In some embodiments, the at least one input mechanism 328 may include a speed change control button. In some embodiments, the speed change control button may be built into and/or may be integrated into the at least one input mechanism 328. For example, in some embodiments, pressing inward on the at least one input mechanism 328 toward the handle 312 may actuate the speed change control button. The speed change control button may actuate and/or cycle through a plurality of speed settings to permit at least some degree of customization over movement of the distal tip 316. In some embodiments, the plurality of speed settings may include at least a high-speed setting which permits faster control and/or faster movement of the distal tip 316 and a low speed setting which permits finer control and/or slower movement of the distal tip 316. In some embodiments, the plurality of speed settings may further include a medium speed setting and/or other speed settings (e.g., medium-high, medium low, extra low, etc.).

In some embodiments, the controller 400, the speed change control feature, the speed change control button, and/or the plurality of speed settings may be user customizable. In some embodiments, the user may be able to set speed mode(s), select and/or set desired speed(s), and/or set or save the speed mode(s) and/or desired speed(s). In some embodiments, the speed change control button may be depressed and held to set or save the speed mode(s) and/or desired speed(s). In some embodiments, the controller 400, the handle 312, and/or the user interface may include additional buttons, a keypad, a scroll wheel, a dial, a touch interface, or other input mechanism for setting and/or adjusting the speed mode(s) and/or desired speed(s). These are only examples, and other configurations are also contemplated.

In some embodiments, the at least one input mechanism 328 may include other control mechanisms. In some embodiments, the at least one input mechanism 328 may include a keypad. In some embodiments, the at least one input mechanism 328 may include a scroll wheel or multiple scroll wheels. In some embodiments, the at least one input mechanism 328 may include a touch screen. Other control configurations are also contemplated.

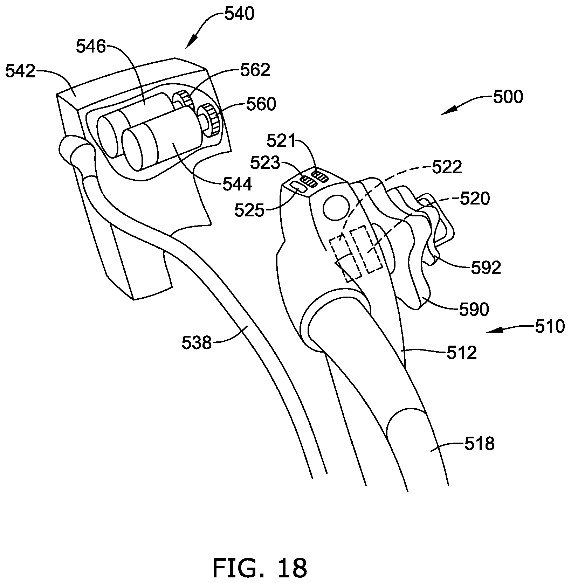

FIG. 18 illustrates an alternative configuration of a medical device, depicted as an endoscopic system 500. In some embodiments, the endoscopic system 500 may include an endoscope 510. The endoscope 510 may be specific to a particular endoscopic procedure, such as, e.g., a ureteroscope, a cystoscope, a nephroscope, a duodenoscope, etc., or may be a general-purpose device suitable for a wide variety of procedures. In some embodiments, the endoscope 510 includes a handle 512 and an elongate shaft (not shown) extending distally from the handle 512 to a distal tip (not shown), wherein the distal tip may be similar in form and/or function to the distal tip 116 described herein. The handle 512 may include a first deflection mechanism 520 configured to deflect and/or articulate the distal tip of the elongate shaft in a first plane and a second deflection mechanism 522 configured to deflect and/or articulate the distal tip of the elongate shaft in a second plane different from the first plane, wherein the first deflection mechanism 520 may be similar in form and/or function to the first deflection mechanism 120 described herein and the second deflection mechanism 522 may be similar in form and/or function to the second deflection mechanism 122 described herein. In some embodiments, the first plane may be oriented at a non-zero angle to the second plane. In some embodiments, the first plane may be oriented perpendicular to the second plane. Other configurations are also contemplated. Although depicted as an endoscopic system 500 including an endoscope 510, it is noted and understood that features, components, and/or functionality described herein may be incorporated into another medical device, such as a steerable catheter or other medical device having one or more deflection mechanisms for controlling deflection and/or articulation of a distal tip of the elongate shaft of the medical device to facilitate navigation of the elongate shaft through the anatomy of a patient. Accordingly, the described components such as the handle, elongate shaft, deflection mechanisms, motor control assembly, and other components may be associated with another medical device having a deflectable/steerable distal tip of an elongate shaft, as desired.

In some embodiments, the first deflection mechanism 520 may include a first pulley or rotating member disposed within the handle 512 and operatively connected to the distal tip. In some embodiments, one or more first cables, wires, or other filaments may be engaged with and/or connected to the first pulley within the handle 512. In some embodiments, the one or more first cables, wires, or filaments may be engaged with and/or connected to the distal tip, such that tension applied to the one or more first cables, wires, or filaments by the first pulley deflects and/or articulates the distal tip in the first plane. In some embodiments, the second deflection mechanism 522 may include a second pulley or rotating member disposed within the handle 512 and operatively connected to the distal tip. In some embodiments, one or more second cables, wires, or other filaments may be engaged with and/or connected to the second pulley within the handle 512. In some embodiments, the one or more second cables, wires, or filaments may be engaged with and/or connected to the distal tip, such that tension applied to the one or more second cables, wires, or filaments by the second pulley deflects and/or articulates the distal tip in the second plane. Other configurations are also contemplated.

In some embodiments, the endoscopic system 500 may include a motor control assembly 540 including a motor control housing 542 configured to detachably interface with the handle 512 of the endoscope 510. In FIG. 18, the motor control housing 542 is shown in a partial cutaway view. In some embodiments, the motor control housing 542 may include a removable cover, wherein internal components of the motor control assembly 540 may be accessed after removing the removable cover. Additionally, in some embodiments, the motor control assembly 540 may include one or more internal covers disposed within the motor control housing 542 that may protect certain components and/or groups of components from each other, contamination, etc. In some embodiments, the one or more internal covers may provide structural support for selected internal components. It shall be understood that the presence and/or use of all, some, or any of the one or more internal covers is optional and is not required.

In some embodiments, the motor control assembly 540 may include at least one motor disposed within the motor control housing 542. In some embodiments, the at least one motor may include a first motor 544 disposed within the motor control housing 542. In some embodiments, the at least one motor may include a second motor 546 disposed within the motor control housing 542. In some embodiments, the at least one motor may include the first motor 544 disposed within the motor control housing 542 and the second motor 546 disposed within the motor control housing 542. In some embodiments, for each controlled degree of freedom of the distal tip, an individual motor may be added to the at least one motor.

The at least one motor may be electric and may be brushed or brushless DC motors. The at least one motor may provide the user partial force assistance for deflecting the distal tip, from 0% to 100% force assistance, thereby allowing for fully manual control, assisted control with reduced lever/knob force, or fully actuated control. Each motor may be attached to a rotary encoder which provides a relative position of said motor to the processor unit. In some embodiments, the at least one motor may be controller by a central processing unit (CPU), a microprocessor, and/or a combination thereof. In some embodiments, additional inputs for the CPU, the microprocessor, and/or the combination thereof may include components of the user interface, tactile feedback electronics, etc.

In some embodiments, the handle 512 may include a user input mechanism. In some embodiments, the user input mechanism may include one or more of a joystick control, a scroll wheel, a keypad, a touch screen interface or control, a voice interface or control, etc. As illustrated, the handle 512 may include a first knob 590 coupled to and/or configured to operate the first deflection mechanism 520. In some embodiments, the handle 512 may include a second knob 592 configured to operate the second deflection mechanism 522.

In some embodiments, the first knob 590 and/or the second knob 592 may be configured to control movement of the distal tip, thus providing the user with the haptic feel and visual cues of a fully manual endoscope. However, as discussed above, the motor control assembly 540 may provide at least partial force assistance to the first knob 590 and/or the second knob 592 for moving the distal tip. The handle 512 may include a torque sensor, a position sensor, an encoder, etc. associated with the first knob 590 and/or the first deflection mechanism 520 for detecting changes in position and/or desired movement of the distal tip in the first plane, and the handle 512 may include a torque sensor, a position sensor, an encoder, etc. associated with the second knob 592 and/or the second deflection mechanism 522 for detecting changes in position and/or desired movement of the distal tip in the second plane.

The first motor 544 may be configured to assist the first deflection mechanism 520 when the first knob 590 is actuated, rotated, turned, activated, etc. The second motor 546 may be configured to assist the second deflection mechanism 522 when the second knob 592 is actuated, rotated, turned, activated, etc. As such, the first knob 590 may be configured to indirectly operate the first motor 544. The second knob 592 may be configured to indirectly operate the second motor 546. In some embodiments, the first knob 590 and the second knob 592 may be configured to indirectly operate the first motor 544 and the second motor 546 simultaneously. In some embodiments, the first knob 590 and the second knob 592 may be configured to indirectly operate the first motor 544 and the second motor 546 independently of each other. Other configurations are also contemplated. In some instances, the first motor 544 may provide partial force assistance in rotating the first knob 590, and thus reduce the force applied on the knob 590 by the operator to actuate the first deflection mechanism 520 to deflect the distal tip. Similarly, in some instances, the second motor 546 may provide partial force assistance in rotating the second knob 592, and thus reduce the force applied on the knob 592 by the operator to actuate the second deflection mechanism 522 to deflect the distal tip. In some instances, the amount of force assistance provided by the first motor 544 and/or the second motor 546 may be adjusted to provide a desired amount of assistance in operating the deflection mechanisms 520, 522. For example, the motors 544, 546 may reduce the amount of force needed to be applied to the knobs 590, 592 by the operator to effect deflection of the distal tip by 10% or more, 20% or more, 30% or more, 40% or more, or 50% or more, in some instances. In some instances, the first motor 544 may provide substantially full force assistance in rotating the first knob 590, and thus reduce the force applied on the knob 590 by the operator to actuate the first deflection mechanism 520 to deflect the distal tip. Similarly, in some instances, the second motor 546 may provide substantially full force assistance in rotating the second knob 592, and thus reduce the force applied on the knob 592 by the operator to actuate the second deflection mechanism 522 to deflect the distal tip. For example, the motors 544, 546 may reduce the amount of force needed to be applied to the knobs 590, 592 by the operator to effect deflection of the distal tip by 80% or more, 85% or more, 90% or more, or 95% or more, in some instances.

In some embodiments, the endoscopic system 500 may include a first drive gear 560 coupled to the first motor 544, wherein the first drive gear 560 is configured to engage with a first gear 521 coupled to the first deflection mechanism 520 disposed within the handle 512. In some embodiments, the first drive gear 560 may be at least partially disposed within the motor control housing 542. In some embodiments, the first gear 521 may be at least partially disposed within the handle 512. In some embodiments, the endoscopic system 500 may include a second drive gear 562 coupled to the second motor 546, wherein the second drive gear 562 is configured to engage with a second gear 523 coupled to the second deflection mechanism 522 disposed within the handle 512. In some embodiments, the second drive gear 562 may be at least partially disposed within the motor control housing 542. In some embodiments, the second gear 523 may be at least partially disposed within the handle 512. Coupling the motor control housing 542 to the handle 512 may engage the first drive gear 560 with the first gear 521 and may engage the second drive gear 562 with the second gear 523. Thus, rotating the first drive gear 560 by the first motor 544 in turn rotates the first gear 521, which in turn actuates the first deflection mechanism 520 to deflect the distal tip in a first plane. Furthermore, rotating the second drive gear 562 by the second motor 546 in turn rotates the second gear 523, which in turn actuates the second deflection mechanism 522 to deflect the distal tip in a second plane. Other configurations are also contemplated.

In some embodiments, the motor control assembly 540 may include a homing feature configured to return the distal tip to a home position (e.g., to a straightened and/or non-deflected configuration) and/or a speed control feature configured to control speed and/or responsiveness of movement of the distal tip. In some embodiments, the homing feature and/or the speed control feature may each include an input mechanism. For example, the input mechanism may be one or more buttons, a voice interface and/or control, a gesture interface and/or control, or other suitable means of providing input to the motor control assembly 540 and/or the user interface.

In some embodiments, the motor control assembly 540 may include one or more buttons. In some embodiments, the one or more buttons may include a first button (e.g., a home button) configured to return the distal tip to a home position (e.g., to a straightened configuration), wherein activation of the first button automatically actuates the at least one motor to move the distal tip to the home position. In some embodiments, the one or more buttons may include a second button that is user configurable to retain a saved position or configuration, wherein activation of the second button automatically actuates the at least one motor to move the distal tip to the saved position and/or configuration. Additional buttons and/or other configurations are also contemplated. Additionally, while the one or more buttons are discussed herein by way of example, the motor control assembly 540 is not limited to the use of physical buttons and may include other input mechanisms.

In some embodiments, the motor control assembly 540 may include a self-homing feature and/or procedure. In one example, upon connecting the motor control assembly 540 to the handle 512, a homing program and/or algorithm is activated and/or run for the first motor 544 and the second motor 546 (where present) sequentially (e.g., the procedure is done on the first motor 544 and then is done on the second motor 546). In some instances, the homing program and/or algorithm is automatically initiated when the motor control assembly 540 is connected to the handle 512. In other instances, the user may push a button, or otherwise manually initiate the homing program and/or algorithm after the motor control assembly 540 is connected to the handle 512. The first motor 544 may start rotating in a first direction while the current is being monitored by the homing program and/or algorithm. Upon registering, encountering, and/or identifying a sudden increase in the amount of current drawn by the first motor 544, a location of an encoder is saved as a checkpoint for an upper limit or a lower limit. The first motor 544 then starts turning in a second direction opposite the first direction and again upon registering, encountering, and/or identifying a sudden increase in the amount of current drawn by the first motor 544, the value of the encoder is saved as a second checkpoint for the other limit of the motion range for that axis and/or the first motor 544. After determining the upper limit and the lower limit of the first motor 544 (and finding their corresponding values in terms of encoder values) the algorithm may divide the number of ticks between the upper limit and the lower limit by two. The corresponding encoder value would be the middle of the motion range or the home position for that axis and/or the first motor 544.

The same procedure is then repeated for the second motor 546, wherein the second motor 546 may start rotating in the first direction while the current is being monitored by the homing program and/or algorithm. Upon registering, encountering, and/or identifying a sudden increase in the amount of current drawn by the second motor 546, a location of an encoder is saved as a checkpoint for an upper limit or a lower limit. The second motor 546 then starts turning in the second direction opposite the first direction and again upon registering, encountering, and/or identifying a sudden increase in the amount of current drawn by the second motor 546, the value of the encoder is saved as a second checkpoint for the other limit of the motion range for that axis and/or the second motor 546. After determining the upper limit and the lower limit of the second motor 546 (and finding their corresponding values in terms of encoder values) the algorithm may divide the number of ticks between the upper limit and the lower limit by two. The corresponding encoder value would be the middle of the motion range or the home position for that axis and/or the second motor 546.

In some instances, the first and/or second deflection mechanisms 520, 522 may include a sensor for determining the position of the pulley of the first and/or second deflection mechanism 520, 522 and/or angular rotation of the pulley of the first and/or second deflection mechanism 520, 522. By sensing the position and/or angular rotation of the pulley, the amount of displacement of the distal tip may be determined. In other instances, the first and/or second deflection mechanisms 520, 522 may include a sensor for otherwise determining the amount of displacement of the distal tip. The handle 512 of the endoscope 510 may include an electrical interface 525 including one or more, or a plurality of electrical contacts for powering the sensor and/or receiving signals from the sensor. The electrical interface 525 may be configured to mate with a corresponding electrical interface (not shown) on the motor control assembly 540.

In another example, the homing feature and/or procedure may be manually activated after engaging the motor control assembly 540 to the handle 512 of the endoscope 510. Other configurations are also contemplated.

In some embodiments, movement of the first knob 590 and/or the second knob 592 may be configured to control a speed at which the distal tip moves. In some embodiments, the motor control assembly 540 and/or the handle 512 may include a speed change control button. The speed change control button may actuate and/or cycle through a plurality of speed settings to permit at least some degree of customization over movement of the distal tip and/or force assistance provided by the first motor 544 and/or the second motor 546. In some embodiments, the plurality of speed settings may include at least a high-speed setting which permits faster control and/or faster movement of the distal tip and a low speed setting which permits finer control and/or slower movement of the distal tip. In some embodiments, the plurality of speed settings may further include a medium speed setting and/or other speed settings (e.g., medium-high, medium low, extra low, etc.).

In some embodiments, the motor control housing 542, the speed change control feature, the speed change control button, and/or the plurality of speed settings may be user customizable. In some embodiments, the user may be able to set speed mode(s), select and/or set desired speed(s), and/or set or save the speed mode(s) and/or desired speed(s). In some embodiments, the speed change control button may be depressed and held to set or save the speed mode(s) and/or desired speed(s). In some embodiments, the motor control housing 542 and/or the user interface may include additional buttons, a keypad, a scroll wheel, a dial, a touch interface, or other input mechanism for setting and/or adjusting the speed mode(s) and/or desired speed(s). These are only examples, and other configurations are also contemplated.

In some embodiments, the distal tip may include a camera and may, for example, have deflection and/or articulation capabilities in one or more directions for viewing patient anatomy. In some embodiments, the endoscope 510 may be a duodenoscope such as an Exalt^{™} Model D scope. However, other medical devices, such as another endoscope (e.g., a ureteroscope, etc.) or related system, (e.g., Lithovue^{™}, SpyScope^{™} DS, SpyGlass^{™} DS, etc.) may be used in addition to or in place of a duodenoscope and/or the endoscope 510. In some embodiments, the endoscope 510 may be configured to deliver fluid from a fluid management system to a treatment site via the elongate shaft. The elongate shaft may include one or more working lumens for receiving a flow of fluid and/or other medical devices therethrough. In some embodiments, the endoscope 510 may be connected to the fluid management system via one or more supply line(s).

In some embodiments, the handle 512 of the endoscope 510 may include a plurality of elements configured to facilitate the endoscopic procedure. In some embodiments, an umbilicus 518 extends from the handle 512 and is configured for attachment to an electronic device (not pictured) such as e.g. a computer system, a console, a microcontroller, etc. for providing power, analyzing endoscopic data, controlling the endoscopic intervention, and/or performing other functions. In some embodiments, the electronic device to which the umbilicus 518 is connected may have functionality for recognizing and exchanging data with other endoscopic accessories. The handle 512 may include a grip area 524 for the operating physician to grasp while performing the endoscopic procedure. In some embodiments, the handle 512 may include a side port in communication with the one or more working lumens of the elongate shaft and/or the endoscope 510.

In some embodiments, a motor control umbilicus 538 may extend from the motor control housing 542 and may be configured for attachment to an electronic device, which may be the same electronic device that the umbilicus 518 is attached to or may be a different electronic device as desired, for providing power, controlling endoscopic intervention and/or the motor control assembly, and/or other functions. In some embodiments, the motor control umbilicus 538 may be secured to the umbilicus 518. In some embodiments, the motor control umbilicus 538 may be releasably and/or removably secured to the umbilicus 518. In some embodiments, the motor control umbilicus 538 may be secured to the umbilicus 518 with a hook and loop closure device (e.g., Velcro^{™}), tape, a wire tie, or other securement apparatus.

In some embodiments, the endoscope 510 may be in electronic communication with a workstation via a wired connection (e.g., the umbilicus 518). In some embodiments, the workstation may include a touch panel computer, an interface box for receiving the wired connection (e.g., the umbilicus 518), a cart, and a power supply, among other features. In some embodiments, the interface box may be configured with a wired or wireless communication connection with the controller of the fluid management system. The touch panel computer may include at least a display screen and an image processor. In some embodiments, the workstation may be a multi-use component (e.g., used for more than one procedure) while the endoscope 510 may be a single use device, although this is not required. In some embodiments, the workstation may be omitted and the endoscope 510 may be electronically coupled directly to the controller of a fluid management system, where used.

In some embodiments, the handle 512 may include at least one communication interface for attaching accessory devices. In some embodiments, the handle 212 may include Universal Serial Bus type-C (USB-C) ports, Universal Serial Bus (USB) ports, ethernet ports, and/or other types of ports. In some embodiments, more, less, and/or other communication interfaces of various types, including, for example, custom interfaces, may be used. In some embodiments, the handle 512 has only one communication interface but may be connectable to e.g. a USB hub with multiple ports for connecting multiple accessories. In some embodiments, the at least one communication interface may provide power to the accessory device(s) in addition to exchanging data therewith. Thus, the accessory device(s) need not have separate cables running to a connected electronic device or a battery that adds additional weight to the handle 512. In some embodiments, the accessory device(s) may be uniquely associated with the endoscope 510 and recognized by the electronic device through "plug and play" functionality without any user setup required.

In some embodiments, the endoscope 510 may include one or more sensors proximate the distal tip and/or the distal end of the elongate shaft 514. For example, the endoscope 510 may include a pressure sensor at the distal tip of the elongate shaft 514 to measure intracavity pressure within the treatment site. The endoscope 510 may also include other sensors such as, for example, a temperature sensor, a Fiber Bragg grating optical fiber to detect stresses, and/or an antenna or electromagnetic sensor (e.g., a position sensor). In some embodiments, the distal tip and/or the distal end of the endoscope 510 may also include at least one camera to provide a visual feed to the user on the display screen of the touch panel computer. In another embodiment, the endoscope 510 may include two cameras having different communications requirements or protocols so that different information may be relayed to the user by each camera. When so provided, the user may switch back and forth between the cameras at will through the touch screen interface and/or the touch panel computer. While not explicitly shown, the elongate shaft may include one or more working lumens for receiving the fluid and/or other medical devices. In some embodiments, the distal tip may include an elevator configured to manipulate a guidewire, a tool, a medical instrument, etc. extending through the elongate shaft, and the handle 512 may include an elevator control operably connected to the elevator. In some embodiments, the at least one motor may include a motor configured to control and/or power movement of the elevator. In some embodiments, an elevator motor may be disposed within the handle 512 of the endoscope 510.

In some embodiments, the location of the distal tip and/or the distal end of the elongate shaft may be tracked during use. For example, a mapping and navigation system may include an operating table (or other procedural or examination table or chair, etc.) configured to act or function as an electromagnetic generator to generate a magnetic field of a known geometry. Alternatively, or additionally, an electromagnetic generator separate from the operating table may be provided. The operating table and/or the electromagnetic generator may be coupled to a control unit which may include among other features, a processor, a memory, a display, and an input means. A position sensor (e.g., the electromagnetic sensor, etc.) or antenna, may be incorporated into the distal tip and/or the distal end of the elongate shaft of the endoscope 510. The position sensor may be configured for use in sensing a location of the position sensor in the magnetic field of the mapping and navigation system. In some embodiments, the position sensor may be electronically coupled to the workstation. When the position sensor is in the magnetic field, the location of the position sensor can be mathematically determined relative to the electromagnetic field source (e.g., the operating table and/or the electromagnetic generator). The workstation and the control unit may communicate to determine the position of the position sensor relative to the patient.

In some embodiments, the motor control housing 542 may include a securement element attached to, integrated with, and/or extending outward from the motor control housing 542. The securement element may be configured to engage with and/or secure to the handle 512 of the endoscope 510. In some embodiments, the securement element may include a first portion of a twist-lock mechanism and the handle 512 of the endoscope 510 may include a second portion of the twist-lock mechanism, wherein the second portion of the twist-lock mechanism is configured to engage with and/or receive the first portion of the twist-lock mechanism to detachably interface and/or secure the motor control assembly 540 and/or the motor control housing 542 to the handle 512 of the endoscope 210. Other configurations and/or means of securing the motor control assembly 540 and/or the motor control housing 542 to the handle 512 of the endoscope 510 are also contemplated. For example, in some embodiments, the motor control housing 542 may be attached to the handle 512 using mechanical fasteners (e.g., screws, bolts, latches, cam locks, etc.). In some embodiments, the motor control housing 542 may be attached to the handle 512 using one or more magnets. In some embodiments, the handle 512 of the endoscope 510 may include a plurality of spring-loaded plungers configured to bias the motor control housing 542 away from the handle 512 of the endoscope 510 to remove any slack between the motor control housing 542 and the handle 512 of the endoscope 510. In some alternative embodiments, the motor control housing 542 may include a plurality of spring-loaded plungers configured to bias the motor control housing 542 away from the handle 512 of the endoscope 510 to remove any slack between the motor control housing 542 and the handle 512 of the endoscope 510.

In some embodiments, the handle 512 of the endoscope 510 may include a first alignment feature and the motor control housing 542 may include a second alignment feature configured to engage with the first alignment feature when the motor control assembly 540 and/or the motor control housing 542 is detachably engaged with the handle 512 of the endoscope 510. In some embodiments, the first alignment feature may cooperate with the second alignment feature to prevent relative rotation between the handle 512 and the motor control housing 542.

In some embodiments, the endoscope 110/210/310/510 may be a disposable and/or a single-use device. In some embodiments, the motor control assembly 140/240/540 and/or the controller 400 may be reusable with appropriate cleaning and/or disinfecting procedures. In some embodiments, the motor control housing 142/242/542 and/or the controller 400 may be autoclavable and/or sterilizable. In some embodiments, the endoscope 110/210/310/510 may be reusable with appropriate cleaning and/or disinfecting procedures.

In some embodiments, the motor control assembly 140/240/540 and/or the handle 312 of the endoscope 310 may include a tactile vibrator configured to provide tactile feedback to the user. In some embodiments, the tactile vibrator may include an eccentric rotating mass actuator, a piezoelectric actuator, or other suitable elements. Feedback may be artificially created or based on a sensor that measures information arising from physical interaction with its environment. In some embodiments, a graphical representation may be used to provide visual feedback to the user. In some embodiments, an intensity of vibrations may be scaled to a level of electrical current being drawn and/or used by the actuator(s). In some embodiments, the tactile feedback may be used alone or in conjunction with other feedback modalities including audible (e.g., beeping sound) or visual feedback (e.g., warning on user interface, LED lights on motor control housing, etc.) to provide feedback to the user regarding the amount of electrical current drawn by the first motor and/or the second motor that could signal the user regarding reaching the end of the range of motion of the motor(s) and/or a maximum deflection of the distal tip. For example, the amount of electrical current drawn by the first motor and/or the second motor may be used to determine the degree of displacement/deflection of the distal tip. A rapid spike/increase in the sensed electrical current may indicate that the distal tip is approaching and/or at its maximum deflection. In such instances, tactile feedback, audible feedback, and/or visual feedback may be used to inform the user that the distal tip is approaching and/or at its maximum deflection. Additionally, in some embodiments, haptic feedback may be used to warn the user about contact and/or a collision between the distal tip and the patient's anatomy. This may be achieved by monitoring the current drawn by the motor(s) and upon a sudden spike in electrical current usage and/or draw, the handle may start to shake, vibrate, beep, light up, etc. to inform the user of the contact and/or collision of the distal tip with the patient's anatomy.

In some embodiments, the motor control housing 142/242/542 may include a balance weight disposed therein and/or integrated into the motor control housing 142/242/542 to provide balance to the handle 112/212/512 of the endoscope 110/210/510, respectively. Alternatively, in some embodiments, a position of the motor control housing 142/242/542 relative to the handle 112/212/512 of the endoscope 110/210/510, respectively, may be shifted proximally, distally, and/or laterally to change and/or improve balance and/or weight distribution of the combined structure(s).

In some embodiments, the endoscope(s), the motor control assembly (assemblies), and/or the controller described herein may include one or more safety mechanisms configured to prevent tissue damage and/or damage to the endoscope itself. In some embodiments, the endoscope(s) may include on/off indicators (e.g., lights, LEDs, etc.) showing the user whether or not selected features are engaged or disabled. In some embodiments, a strain gauge on the cable, wire, or filament engaged with the pulley, or optical strain gauge fibers, may be used to infer pressure exerted by the endoscope on adjacent tissue. In any case, a safety threshold for pressure or strain may be an adjustable input variable that the user is able to choose and/or adjust before and/or during the procedure. When the pressure or strain exceeds the safety threshold, the distal tip may return to the home position, or other pre-programmed actions may be implemented. In some embodiments, the user may choose between these options as another user input factor. In some embodiments, there may be more than one pressure or strain safety threshold. In some embodiments, the safety threshold(s) may be activated and/or deactivated by users via voice commands, buttons on the endoscope, touchscreen controls, or other methods.

The materials that can be used for the various components of the system(s) and the various elements thereof disclosed herein may include those commonly associated with medical devices. For simplicity purposes, the following discussion refers to the system. However, this is not intended to limit the devices and methods described herein, as the discussion may be applied to other elements, members, components, or devices disclosed herein, such as, but not limited to, the handle(s), the elongate shaft(s), the deflection mechanism(s), the motor(s), the pulley(s), the drive axle(s), the joystick control(s), the button(s), the distal tip(s), the motor control housing(s), etc., and/or elements or components thereof.

In some embodiments, the system, and/or components thereof, may be made from a metal, metal alloy, polymer (some examples of which are disclosed below), a metal-polymer composite, ceramics, combinations thereof, and the like, or other suitable material.

Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), MARLEX^{®} high-density polyethylene, MARLEX^{®} low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, polyurethane silicone copolymers (for example, ElastEon^{®} from Aortech Biomaterials or ChronoSil^{®} from AdvanSource Biomaterials), biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY ^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; platinum; palladium; gold; combinations thereof; or any other suitable material.

In at least some embodiments, portions or all of the system, and/or components thereof, may also be doped with, made of, or otherwise include a radiopaque material. Radiopaque materials are understood to be materials capable of producing a relatively bright image on a fluoroscopy screen or another imaging technique during a medical procedure. This relatively bright image aids the user of the system in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the system to achieve the same result.

In some embodiments, a degree of Magnetic Resonance Imaging (MRI) compatibility is imparted into the system and/or other elements disclosed herein. For example, the system, and/or components or portions thereof, may be made of a material that does not substantially distort the image and create substantial artifacts (i.e., gaps in the image). Certain ferromagnetic materials, for example, may not be suitable because they may create artifacts in an MRI image. The system, or portions thereof, may also be made from a material that the MRI machine can image. Some materials that exhibit these characteristics include, for example, tungsten, cobalt-chromium-molybdenum alloys (e.g., UNS: R30003 such as ELGILOY^{®}, PHYNOX^{®}, and the like), nickel-cobalt-chromium-molybdenum alloys (e.g., UNS: R30035 such as MP35-N^{®} and the like), nitinol, and the like, and others.

In some embodiments, the system and/or other elements disclosed herein may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vasoactive mechanisms.

It should be understood that this disclosure is, in many respects, only illustrative. Changes may be made in details, particularly in matters of shape, size, and arrangement of steps without exceeding the scope of the present disclosure. This may include, to the extent that it is appropriate, the use of any of the features of one example embodiment being used in other embodiments. The scope of the present disclosure is, of course, defined in the language in which the appended claims are expressed.

## Claims

1. A steerable medical device (110, 210), comprising:
a handle (112, 212) and an elongate shaft (114, 214) extending distally from the handle (112, 212) to a distal tip;
a first deflection mechanism (120, 220) disposed within the handle (112, 212), the first deflection mechanism (120, 220) being configured to deflect the distal tip (116, 216) in a first plane;
a motor control assembly (140, 240) including a motor control housing (142, 242) configured to detachably interface with the handle (112, 212);
wherein the motor control assembly (140, 240) includes a first motor (144, 244) disposed within the motor control housing (142, 242) and a first gear assembly (150, 250) disposed within the motor control housing (142, 242); and
a first drive axle (170, 270) configured to engage the first gear assembly (150, 250) with the first deflection mechanism (120, 220),
**characterized in that** the first gear assembly (150, 250) includes a first worm gear shaft 152, 252) and a first worm gear (154, 254), the first worm gear shaft (152, 252) being configured to engage the first worm gear (154, 254).

2. The steerable medical device (110, 210) of claim 1, wherein the motor control assembly (140, 240) further includes a second motor (146, 246) disposed within the motor control housing (142, 242) and a second gear assembly (160, 260) disposed within the motor control housing (142, 242).

3. The steerable medical device (110, 210) of claim 2, further including a second drive axle (180, 280) configured to engage the second gear assembly (160, 260) with a second deflection mechanism (122, 222) disposed within the handle, the second deflection mechanism (122, 222) being configured to deflect the distal tip (116, 216) in a second plane different from the first plane.

4. The steerable medical device (110, 210) of claim 3, wherein the first drive axle (170, 270) is coaxial with the second drive axle (180, 280).

5. The steerable medical device (110, 210) of claim 3, wherein the first drive axle (170, 270) is parallel with the second drive axle (180, 280).

6. The steerable medical device (110, 210) of claim 5, wherein the first drive axle (170, 270) is laterally offset from the second drive axle (180, 280).

7. The steerable medical device (110, 210) of any one of claims 1-6, wherein the motor control assembly (140, 240) includes a joystick control (190, 290) configured to operate the first motor (144, 244).

8. The steerable medical device (110, 210) of any one of claims 2-6, wherein the motor control assembly (140, 240) includes a joystick control (190, 290) configured to operate the first motor (144, 244) and the second motor (146, 246).

9. The steerable medical device (110, 210) of any one of claims 1-8, further comprising a homing feature configured to return the distal tip (116, 216) to a home position when activated and/or a speed control feature configured to control speed and/or responsiveness of movement of the distal tip (116, 216).

10. The steerable medical device (110, 210) of claim 4, wherein the first drive axle (170, 270) is fixedly secured to the first deflection mechanism (120, 220), and wherein the first drive axle (170, 270) is configured to slidably engage the first gear assembly (150, 250);
wherein the second drive axle (180, 280) is fixedly secured to the second deflection mechanism (122, 222), and wherein the second drive axle (180, 280) is configured to slidably engage the second gear assembly (160, 260).

11. The steerable medical device (110, 210) of claim 6, wherein the first drive axle (170, 270) is fixedly secured to the first gear assembly (150, 250), and wherein the first drive axle (170, 270) is configured to slidably engage the first deflection mechanism (120, 220);
wherein the second drive axle (180, 280) is fixedly secured to the second gear assembly (160, 260), and wherein the second drive axle (180, 280) is configured to slidably engage the second deflection mechanism (122, 222).

## Patentansprüche

1. Lenkbare medizinische Vorrichtung (110, 210), aufweisend:
einen Griff (112, 212) und einen länglichen Schaft (114, 214), der sich von dem Griff (112, 212) zu einer distalen Spitze distal erstreckt;
einen ersten Ablenkmechanismus (120, 220), der innerhalb des Griffs (112, 212) angebracht ist, wobei der erste Ablenkmechanismus (120, 220) so konfiguriert ist, dass er die distale Spitze (116, 216) in einer ersten Ebene ablenkt;
eine Motorsteuerungsanordnung (140, 240), die ein Motorsteuerungsgehäuse (142, 242) aufweist, das so konfiguriert ist, dass es mit dem Griff (112, 212) lösbar zusammengefügt ist;
wobei die Motorsteuerungsanordnung (140, 240) einen ersten Motor (144, 244), der innerhalb des Motorsteuerungsgehäuses (142, 242) angebracht ist, und eine erste Getriebeanordnung (150, 250) aufweist, die innerhalb des Motorsteuerungsgehäuses (142, 242) angebracht ist; und
eine erste Antriebsachse (170, 270), die so konfiguriert ist, dass sie die erste Getriebeanordnung (150, 250) mit dem ersten Ablenkmechanismus (120, 220) in Eingriff bringt,
**dadurch gekennzeichnet, dass**
die erste Getriebeanordnung (150, 250) eine erste Schneckenradwelle (152, 252) und ein erstes Schneckenrad (154, 254) aufweist, wobei die erste Schneckenradwelle (152, 252) so konfiguriert ist, dass sie mit dem ersten Schneckenrad (154, 254) in Eingriff kommt.

2. Lenkbare medizinische Vorrichtung (110, 210) nach Anspruch 1, wobei die Motorsteuerungsanordnung (140, 240) ferner einen zweiten Motor (146, 246), der innerhalb des Motorsteuerungsgehäuses (142, 242) angebracht ist, und eine zweite Getriebeanordnung (160, 260) aufweist, die innerhalb des Motorsteuerungsgehäuses (142, 242) angebracht ist.

3. Lenkbare medizinische Vorrichtung (110, 210) nach Anspruch 2, ferner aufweisend eine zweite Antriebsachse (180, 280), die so konfiguriert ist, dass sie die zweite Getriebeanordnung (160, 260) mit einem zweiten Ablenkmechanismus (122, 222) in Eingriff kommt, der innerhalb des Griffs angebracht ist, wobei der zweite Ablenkmechanismus (122, 222) so konfiguriert ist, dass er die distale Spitze (116, 216) in einer zweiten Ebene ablenkt, die sich von der ersten Ebene unterscheidet.

4. Lenkbare medizinische Vorrichtung (110, 210) nach Anspruch 3, wobei die erste Antriebsachse (170, 270) mit der zweiten Antriebsachse (180, 280) koaxial ist.

5. Lenkbare medizinische Vorrichtung (110, 210) nach Anspruch 3, wobei die erste Antriebsachse (170, 270) parallel zu der zweiten Antriebsachse (180, 280) liegt.

6. Lenkbare medizinische Vorrichtung (110, 210) nach Anspruch 5, wobei die erste Antriebsachse (170, 270) von der zweiten Antriebsachse (180, 280) seitlich versetzt ist.

7. Lenkbare medizinische Vorrichtung (110, 210) nach einem der Ansprüche 1-6, wobei die Motorsteuerungsanordnung (140, 240) eine Joystick-Steuerung (190, 290) aufweist, die so konfiguriert ist, dass sie den ersten Motor (144, 244) betreibt.

8. Lenkbare medizinische Vorrichtung (110, 210) nach einem der Ansprüche 2-6, wobei die Motorsteuerungsanordnung (140, 240) eine Joystick-Steuerung (190, 290) aufweist, die so konfiguriert ist, dass sie den ersten Motor (144, 244) und den zweiten Motor (146, 246) betreibt.

9. Lenkbare medizinische Vorrichtung (110, 210) nach einem der Ansprüche 1-8, ferner aufweisend eine Rückkehrfunktion, die so konfiguriert ist, dass die distale Spitze (116, 216) in eine Ausgangsposition zurückkehrt, wenn sie aktiviert wird, und/oder eine Geschwindigkeitssteuerfunktion, die so konfiguriert ist, dass sie die Geschwindigkeit und/oder die Reaktionsfähigkeit der Bewegung der distalen Spitze (116, 216) steuert.

10. Lenkbare medizinische Vorrichtung (110, 210) nach Anspruch 4, wobei die erste Antriebsachse (170, 270) an dem ersten Ablenkmechanismus (120, 220) fest angebracht ist und wobei die erste Antriebsachse (170, 270) so konfiguriert ist, dass sie in die erste Getriebeanordnung (150, 250) gleitend eingreift;
wobei die zweite Antriebsachse (180, 280) an dem zweiten Ablenkmechanismus (122, 222) fest angebracht ist und wobei die zweite Antriebsachse (180, 280) so konfiguriert ist, dass sie in die zweite Getriebeanordnung (160, 260) gleitend eingreift.

11. Lenkbare medizinische Vorrichtung (110, 210) nach Anspruch 6, wobei die erste Antriebsachse (170, 270) an der ersten Getriebeanordnung (150, 250) fest angebracht ist und wobei die erste Antriebsachse (170, 270) so konfiguriert ist, dass sie in den ersten Ablenkmechanismus (120, 220) gleitend eingreift;
wobei die zweite Antriebsachse (180, 280) an der zweiten Getriebeanordnung (160, 260) fest angebracht ist und wobei die zweite Antriebsachse (180, 280) so konfiguriert ist, dass sie in den zweiten Ablenkmechanismus (122, 222) gleitend eingreift.

## Revendications

1. Dispositif médical orientable (110, 210) comprenant :
une poignée (112, 212) et une tige allongée (114, 214) s'étendant de manière distale à partir de la poignée (112, 212) jusqu'à une pointe distale ;
un premier mécanisme de déviation (120, 220) disposé à l'intérieur de la poignée (112, 212), le premier mécanisme de déviation (120, 220) étant configuré pour dévier la pointe distale (116, 216) dans un premier plan ;
un ensemble de commande de moteur (140, 240) comprenant un boîtier de commande de moteur (142, 242) configuré pour s'interfacer, de manière détachable, avec la poignée (112, 212) ;
dans lequel l'ensemble de commande de moteur (140, 240) comprend un premier moteur (144, 244) disposé à l'intérieur du boîtier de commande de moteur (142, 242) et un premier ensemble d'engrenage (150, 250) disposé à l'intérieur du boîtier de commande de moteur (142, 242) ; et
un premier essieu d'entraînement (170, 270) configuré pour mettre en prise un premier ensemble d'engrenage (150, 250) avec le premier mécanisme de déviation (120, 220),
**caractérisé en ce que** :
le premier ensemble d'engrenage (150, 250) comprend un premier arbre d'engrenage à vis sans fin (152, 252) et un premier engrenage à vis sans fin (154, 254), le premier arbre d'engrenage à vis sans fin (152, 252) étant configuré pour mettre en prise le premier engrenage à vis sans fin (154, 254).

2. Dispositif médical orientable (110, 210) selon la revendication 1, dans lequel l'ensemble de commande de moteur (140, 240) comprend en outre un deuxième moteur (146, 246) disposé à l'intérieur du boîtier de commande de moteur (142, 242) et un deuxième ensemble d'engrenage (160, 260) disposé à l'intérieur du boîtier de commande de moteur (142, 242).

3. Dispositif médical orientable (110, 210) selon la revendication 2, comprenant en outre un deuxième essieu d'entraînement (180, 280) configuré pour mettre en prise le deuxième ensemble d'engrenage (160, 260) avec un deuxième mécanisme de déviation (122, 222) disposé à l'intérieur de la poignée, le deuxième mécanisme de déviation (122, 222) étant configuré pour dévier la pointe distale (116, 216) dans un deuxième plan différent du premier plan.

4. Dispositif médical orientable (110, 210) selon la revendication 3, dans lequel le premier essieu d'entraînement (170, 270) est coaxial par rapport au deuxième essieu d'entraînement (180, 280).

5. Dispositif médical orientable (110, 210) selon la revendication 3, dans lequel le premier essieu d'entraînement (170, 270) est parallèle au deuxième essieu d'entraînement (180, 280).

6. Dispositif médical orientable (110, 210) selon la revendication 5, dans lequel le premier essieu d'entraînement (170, 270) est latéralement décalé du deuxième essieu d'entraînement (180, 280).

7. Dispositif médical orientable (110, 210) selon l'une quelconque des revendications 1 à 6, dans lequel l'ensemble de commande de moteur (140, 240) comprend une commande par manette (190, 290) configurée pour actionner le premier moteur (144, 244).

8. Dispositif médical orientable (110, 210) selon l'une quelconque des revendications 2 à 6, dans lequel l'ensemble de commande de moteur (140, 240) comprend une commande par manette (190, 290) configurée pour actionner le premier moteur (144, 244) et le deuxième moteur (146, 246).

9. Dispositif médical orientable (110, 210) selon l'une quelconque des revendications 1 à 8, comprenant en outre une caractéristique de guidage configurée pour ramener la pointe distale (116, 216) dans une position initiale, lorsqu'elle est activée et/ou une caractéristique de contrôle de vitesse configurée pour contrôler la vitesse et/ou la réactivité de mouvement de la pointe distale (116, 216).

10. Dispositif médical orientable (110, 210) selon la revendication 4, dans lequel le premier essieu d'entraînement (170, 270) est fixé, de manière fixe, au premier mécanisme de déviation (120, 220), et dans lequel le premier essieu d'entraînement (170, 270) est configuré pour mettre en prise, de manière coulissante, le premier ensemble d'engrenage (150, 250) ;
dans lequel le deuxième essieu d'entraînement (180, 280) est fixé, de manière fixe, au deuxième mécanisme de déviation (122, 222) et dans lequel le deuxième essieu d'entraînement (180, 280) est configuré pour mettre en prise, de manière coulissante, le deuxième ensemble d'engrenage (160, 260).

11. Dispositif médical orientable (110, 210) selon la revendication 6, dans lequel le premier essieu d'entraînement (170, 270) est fixé, de manière fixe, au premier ensemble d'engrenage (150, 250) et dans lequel le premier essieu d'entraînement (170, 270) est configuré pour mettre en prise, de manière coulissante, le premier mécanisme de déviation (120, 220) ;
dans lequel le deuxième essieu d'entraînement (180, 280) est fixé, de manière fixe, au deuxième ensemble d'engrenage (160, 260) et dans lequel le deuxième essieu d'entraînement (180, 280) est configuré pour mettre en prise, de manière coulissante, le deuxième mécanisme de déviation (122, 222).
